# EUROPÄISCHE PATENTSCHRIFT

(11) **EP 2 287 158 B1**
(45) Veröffentlichungstag und Bekanntmachung des Hinweises auf die Patenterteilung: **14.11.2012**
(21) Anmeldenummer: 10181614.8
(22) Anmeldetag: 29.09.2010
(51) Int. Cl.: C07D 317/70, C11B 9/00

(54) **Ambra-Riechstoff auf Basis von 9-Methanoazuleno(5,6-d)-1,3-dioxolen**
Ambergris Odorous Substance based on 9-Methanoazuleno(5,6-d)-1,3-dioxoles
Parfum d'ambre à base de 9-Methanoazuleno(5,6-d)-1,3-dioxoles

(43) Veröffentlichungstag der Anmeldung: 23.02.2011
(73) Patentinhaber: Symrise AG, 37603 Holzminden (DE)
(72) Erfinder: Dilk, Erich, 37603, Holzminden (DE); Eh, Marcus, 37603, Holzminden (DE); Surburg, Horst, 37603, Holzminden (DE)
(74) Vertreter: Eisenführ, Speiser & Partner

(56) Entgegenhaltungen:
- EP-A1- 0 857 723

## Beschreibung

Die vorliegende Erfindung betrifft primär die neue Verbindung der Formel (II) sowie bestimmte Mischungen umfassend die Verbindung der Formel (II) und deren Verwendung als Riechstoff. Daneben betrifft die Erfindung Riechstoffmischungen und parfümierte Produkte umfassend eine (vorzugsweise sensorisch wirksame) Menge der Verbindung der Formel (II) bzw. einer erfindungsgemäßen Mischung. Weitere Aspekte der Erfindung ergeben sich aus der nachfolgenden Beschreibung und den beigefügten Patentansprüchen.

Cedran-Derivate der Formel (A) werden allgemein in EP 857 723 A1 beschrieben und deren Geruchseigenschaften angegeben. Danach besitzen Verbindungen dieser Stoffklasse einen Geruch vom Ambratyp und vermitteln gleichzeitig einen strahlenden, starken Effekt, der ganz unterschiedliche Parfümnoten verstärkt und ihre Duftwirkung verlängert. Für die in den Beispielen 5-17 angegebenen Verbindungen werden jedoch keine konkreten Geruchsbeschreibungen vorgenommen.

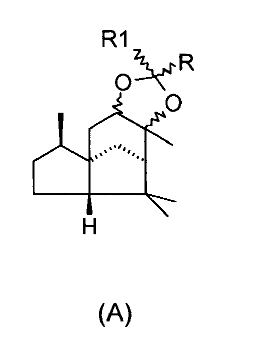

Die geschlängelten Linien können dabei jeweils unabhängig voneinander alpha- oder beta-Konfiguration bedeuten.

Für Verbindungen, in denen R von R¹ verschieden ist werden keine Aussagen zu den Geruchseigenschaften der jeweiligen Stereoisomere gemacht. In EP 857 723 A1 heisst es lediglich, dass die Acetale der Formel (A) jeweils in reiner Form als auch als Stereoisomerengemisch originelle Riechstoffeigenschaften besitzen.

Die Verbindung der Formel (A1) [(4aR,5R,7aS,9R)-Octahydro-2,2,5,8,8,9a-hexamethyl-4H-4a,9-Methanoazuleno(5,6-d)-1,3-dioxol, CAS Nr. 211299-54-6; nachfolgend: Ambrocenide^{®}] besitzt die folgende Struktur, siehe auch EP 0 857 723 A1:

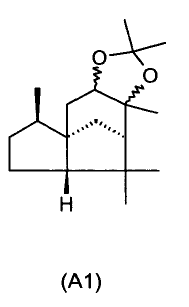

Die geschlängelten Linien können dabei unabhängig voneinander alpha- oder beta-Konfiguration bedeuten. Ambrocenide^{®} der Formel (A1) kann ein, zwei, drei oder sämtliche der folgenden Diastereoisomere umfassen:

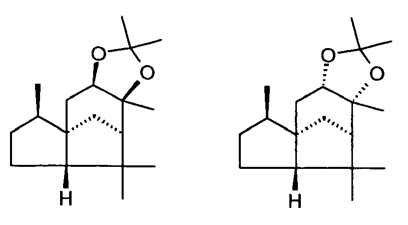

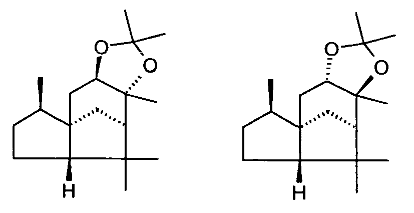

Für die Verwendung einer Substanz als Riechstoff sind neben einem interessanten Geruchsprofil auch noch andere Eigenschaften des Stoffes wie z.B. die Stabilität, die Kompatibilität mit anderen Riechstoffen, die Löslichkeit, sowie die toxikologische Unbedenklichkeit wichtig.

Weitere interessante Aspekte sind ferner das Haftvermögen bzw. die Substantivität eines Riechstoffes, insbesondere auf Fasern und/oder Haaren. Von besonderer Bedeutung ist diese Eigenschaft für tensidhaltige Produkte wie Shampoos, Waschmittel und Weichspüler.

Ambrocenide^{®} ist ein interessanter Riechstoff mit ausgeprägtem Ambra-Geruch und bemerkenswert starker Eigenhaftung.

Gesucht werden, insbesondere für die Parfümierung von tensidhaltigen Formulierungen, Ambra-Riechstoffe mit einer starken Eigenhaftung. Vorzugsweise sollen die Ambra-Riechstoffe wertvolle Geruchsnoten aufweisen. In diesem Zusammenhang besonders gefragt ist die Geruchsnote von weisser Ambra ("white amber"). Unter "white amber" versteht man parfümistisch den Geruch von gealterter natürlicher Ambra, die eine sehr wertvolle Geruchsnote darstellt.

Es war daher die Aufgabe der vorliegenden Erfindung, eine Substanz (Mischung oder Einzelverbindung) mit Ambra-Geruch und besonders starker Eigenhaftung anzugeben, die vorzugsweise eine Geruchsnote von weisser Ambra aufweist.

Diese Aufgabe wird erfindungsgemäß gelöst durch die Verbindung der nachfolgenden Formel (II) sowie bestimmte Mischungen umfassend Verbindung (II) und Verbindung (I).

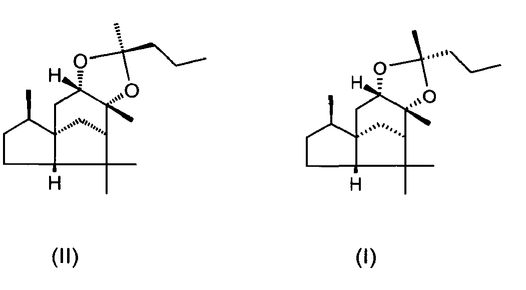

Überraschenderweise wurde gefunden, dass das Stereoisomer der Formel (II) diese Aufgabe erfüllt. Die Verbindung der Formel (II) zeichnet sich durch einen starken Geruch nach Ambra und Holz aus.

Bei der geruchlichen Evaluierung der stereoisomeren Verbindungen der Formeln (I) und (II) erwies sich überraschenderweise nur die erfindungsgemäße Verbindung der Formel (II) als geruchlich interessant, während die isomere Verbindung der Formel (I) lediglich einen sehr schwachen Geruch aufwies.

Die Verbindung der Formel (II) kann dabei sowohl alleine oder in Mischung mit der Verbindung der Formel (I) als Riechstoff verwendet werden.

Bei der geruchlichen Evaluierung der Verbindung der Formel (II) zeigte sich ferner, dass diese Verbindung über eine außergewöhnlich lange Haftung verfügt, die sogar die von Ambrocenide^{®} übertrifft.

Aus cis-Cedrandiol und Methyl-ethyl-keton (2-Butanon) wurden die zu den Verbindungen (I) und (II) strukturell verwandten Acetale der Formeln (Z-1) und (Z-2) erhalten.

Die Verbindung der Formel (Z-2) riecht deutlich stärker als die isomere Verbindung der Formel (Z-1).

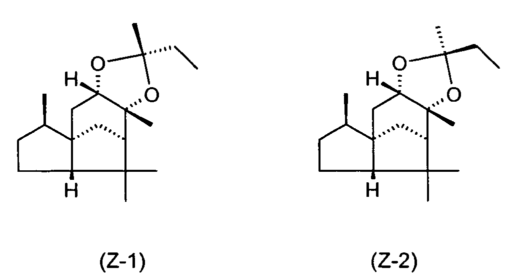

Ein geruchlicher Vergleich der Verbindungen der Formel (II) und (Z-2) zeigte, dass die erfindungsgemäße Verbindung der Formel (II) einen deutlich stärkeren Geruch aufweist

Die Erfindung betrifft auch eine Mischung, umfassend
(i) Verbindung der Formel (I)
   und
(ii) Verbindung der Formel (II),
   dadurch gekennzeichnet, dass
   - das Gewichtsverhältnis der Verbindung der Formel (II) zu der Verbindung der Formel (I) im Bereich von 4 : 1 bis 1 : 10 liegt, vorzugsweise im Bereich von 2 : 1 bis 1 : 6, bevorzugt im Bereich von 1 : 1 bis 1 : 3,
   und/oder
   - der Gesamtanteil der Verbindungen der Formeln (I) und (II) zusammen größer ist als 40 Gew.-%, vorzugsweise größer als 55 Gew.-%, bevorzugt größer als 70 Gew.-%, weiter bevorzugt größer als 85 Gew.-%, besonders bevorzugt größer als 95 Gew.-%, bezogen auf die Gesamtmenge der in der Mischung enthaltenen 3,6,8,8-Tetramethyloctahydro-3a,7-methano-azulen-5,6-diol-methyl-n-propylketale.

Die erfindungsgemäße Verbindung der Formel (II) kann mittels Umsetzung von cis-Cedrandiol (Verbindung der Formel (B), welche aus (-)-α-Cedren herstellbar ist), mit Methyl-n-propyl-keton (2-Pentanon, Verbindung der Formel (C)) hergestellt werden.

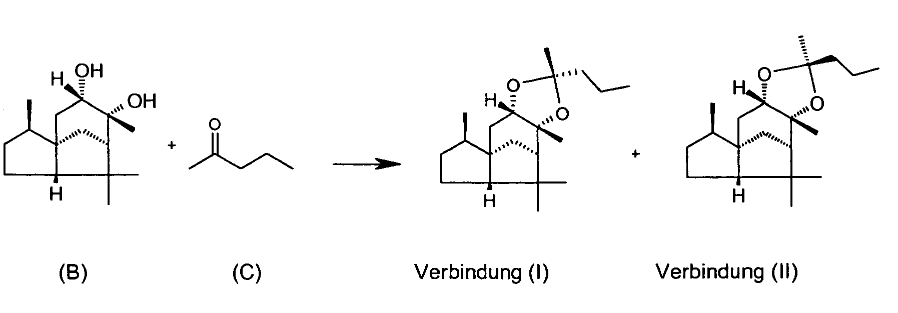

Die erfindungsgemäße Verbindung der Formel (II) bzw. eine erfindungsgemäße Mischung (wie oben definiert) kann hergestellt werden durch Umsetzung von cis-Cedrandiol (Formel (B)) mit Methyl-n-propyl-keton (2-Pentanon) in Gegenwart einer oder mehrerer wasserbindender Substanzen und einer oder mehrerer Säuren.

Bevorzugte wasserbindende Substanzen sind ausgewählt aus der Gruppe bestehend aus Orthoameisensäureestern (vorzugsweise Orthoameisensäuretrimethylester oder Orthoameisensäuretriethylester) und 2,2-Dialkoxypentanen (vorzugswei s e 2,2-Dimethoxypentan oder 2,2-Diethoxypentan).

Die eingesetzten Säuren sind vorzugsweise ausgewählt aus der Gruppe bestehend aus anorganischen Protonensäuren, organischen Protonensäuren und sauren Festbettkatalysatoren.

Vorzugsweise erfolgt die Umsetzung von cis-Cedrandiol (Formel (B)) mit Methyl-n-propyl-keton (2-Pentanon) zu der erfindungsgemäßen Verbindung der Formel (II) bzw. einer erfindungsgemäßen Mischung (wie oben definiert) bei einer Reaktionstemperatur im Bereich von 0 bis 70°C.

Die erfindungsgemäße Verbindung der Formel (II) bzw. eine erfindungsgemäße Mischung (wie oben definiert) zeichnen sich durch ein hohes Aufziehvermögen (Eigenhaftung auf einem Substrat) und eine hohe Substantivität (Fähigkeit, aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern).

Eine weitere wichtige anwendungstechnische Anforderung an Riechstoffe und Riechstoffmischungen, insbesondere für tensidhaltige Produkte, ist deren Substantivität gegenüber dem bzw. Retention am Substrat, insbesondere Haaren oder textilen Fasern. Die Begriffe "Substantivität" und "Retention" werden z.B. in EP 1 201 738 A1 ausführlich dargelegt, vergleiche dort die Abschnitte [0004]-[0005]. Gesucht werden generell Riechstoffe mit hoher Substantivität und/oder Retention.

Diesen Erkenntnissen entsprechend betrifft ein weiterer Aspekt der Erfindung insbesondere die Verwendung der erfindungsgemäßen Verbindung der Formel (II) bzw. einer erfindungsgemäßen Mischung (wie oben definiert) als Mittel zum Erhöhen der Substantivität und/oder Retention einer Riechstoffmischung (insbesondere gegenüber bzw. auf Haaren bzw. textilen Fasern), vorzugsweise einer Riechstoffmischung mit fruchtiger, blumiger, und/oder würziger Note.

Neben ihrem hohen Aufziehvermögen zeichnet sich die erfindungsgemäße Verbindung der Formel (II) durch ihre fixierenden Eigenschaften aus, d.h. sie ist ein Fixateur. Als ein Fixateur erhöht die erfindungsgemäße Verbindung der Formel (II) die Haftfestigkeit von anderen Riechstoffen, sei es durch deren Dampfdruckerniedrigung oder geruchliche Verstärkung (z.B. Absenkung des Schwellenwertes). Die Erfindung betrifft daher auch - wie bereits erwähnt - die Verwendung der erfindungsgemäßen Verbindung der Formel (II) oder einer erfindungsgemäßen Mischung (wie oben charakterisiert) als Fixateur.

Die Erfindung betrifft daher ferner die Verwendung der erfindungsgemäßen Verbindung der Formel (II) bzw. einer wie oben definierte erfindungsgemäße Mischung
- als Riechstoff, vorzugsweise als Riechstoff mit den Geruchsnoten Ambra und/oder Holz,
   und/oder
- als Mittel zum Erhöhen der Substantivität und/oder der Retention einer Riechstoffmischung,
   und/oder
- als Fixateur.
   Weiterhin betrifft die Erfindung eine Riechstoffmischung, vorzugsweise ein Parfümöl, umfassend
- die erfindungsgemäße Verbindung der Formel (II) oder eine erfindungsgemäße Mischung (wie oben definiert),
   und
- einen, 2, 3, 4, 5, 6, 7, 8, 9, 10 oder mehr weitere Riechstoffe, die keine 3,6,8,8-Tetramethyl-octahydro-3a,7-methano-azulen-5,6-diol-methyl-n-propylketale sind,
   wobei
   (i) die Menge an Verbindung der Formel (II) ausreicht, eine Ambranote und/oder Holznote, insbesondere eine weisse Ambranote ("white amber") zu vermitteln,
      und/oder
   (ii) der oder die weiteren Riechstoffe eine, mehrere oder sämtliche der Noten fruchtig, blumig, würzig, holzig, moschus und/oder ambriert vermitteln und die Menge der Verbindung der Formel (II) ausreicht, eine oder mehrere dieser Noten zu modifizieren und/oder zu verstärken.
      und/oder
   (iii) die Menge an Verbindung der Formel (II) ausreicht, der Riechstoffmischung einen Eindruck von Volumen, Komplexität, Eleganz und/oder Natürlichkeit zu verleihen,
      und/oder
   (iv) die Menge an Verbindung der Formel (II) ausreicht, um im Vergleich zu einer ansonsten identisch zusammengesetzten Vergleichsriechstoffkomposition ohne Verbindung der Formel (II), einen pflegenderen, harmonischeren, hochwertigeren und/oder natürlicheren Geruchseindruck zu bewirken.

Eine bevorzugte erfindungsgemäße Riechstoffmischung, vorzugsweise ein erfindungsgemäßes Parfümöl, umfasst die Verbindung der Formel (II) in einer Menge im Bereich von 0,0001 bis 25 Gew.-%, besonders bevorzugt im Bereich von 0,001 bis 15 Gew.-%, jeweils bezogen auf das Gesamtgewicht der Riechstoffmischung.

Erfindungsgemäße Riechstoffmischungen, insbesondere Parfümöle, umfassen vorzugsweise zwei, drei, vier, fünf, sechs, sieben, acht, neun, zehn oder mehr Riechstoffe, bevorzugt ausgewählt aus den nachfolgend genannten Stoffen:
Riechstoffe, die in Steffen Arctander, Perfume and Flavor Chemicals, Eigenverlag, Montclair, N. J. 1969; H. Surburg, J. Panten, Common Fragrance and Flavor Materials, 5th Edition, Wiley-VCH, Weinheim 2006 genannt sind.

Als mit der erfindungsgemäßen Verbindung der Formel (II) vorzugsweise kombinierbare Riechstoffe seien insbesondere genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-Citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl, Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-Cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-Tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon bzw. daraus isolierte Inhaltsstoffe;
einzelne Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (*E*,*Z*)-1,3,5-Undecatrien;
aus der Gruppe der aliphatischen Alkohole, wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
aus der Gruppe der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanal-diethylacetal; 1,1-Dimethoxy-2,2,5-trimethyl-4-hexen; Citronellyloxyacetaldehyd;
aus der Gruppe der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on;
aus der Gruppe der aliphatischen schwefelhaltigen Verbindungen wie z. B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
aus der Gruppe der aliphatischen Nitrile wie z. B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
aus der Gruppe der aliphatischen Carbonsäuren und deren Ester wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
aus der Gruppe der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
aus der Gruppe der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
aus der Gruppe der cyclischen Terpenalkohole wie z. B. Menthol; lsopulegol; α-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
aus der Gruppe der cyclischen Terpenaldehyde und -ketone wie z. B. Menthon; Isomenthon; 8-Mercaptomenthan-3-on; Carvon; Campher; Fenchon; a-lonon; β-lonon; αn-Methylionon; β-n-Methylionon; α-lsomethylionon; β-lsomethylionon; a-lron; α-Damascon; β-Damascon; β-Damascenon; γ-Damascon; δ-Damascon; 1-(2,4,4-Trimethyl-2-cyclohexen-1-yl)-2-buten-1-o n ; 1 , 3 , 4 , 6 , 7 , 8 a-Hexahydro-1,1,5,5-tetramethyl-2H-2,4a-methanonaphthalen-8(5H)-on; Nootkaton; Dihydronootkaton; α-Sinensal; β-Sinensal; acetyliertes Cedernholzöl (Methylcedrylketon);
aus der Gruppe der cyclischen Alkohole wie z. B. 4-*tert*-Butylcyclohexanol; 3,3,5-Trimethylcyclohexanol; 3-Isocamphylcyclohexanol; 2,6,9-Trimethyl-(*Z*2,*Z*5,*E*9)-cyclododecatrien-1-ol; 2-lsobutyl-4-methyltetrahydro-2*H*-pyran-4-ol;
aus der Gruppe der cycloaliphatischen Alkohole wie z. B. α,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
aus der Gruppe der cyclischen und cycloaliphatischen Ether wie z. B. Cineol; Cedrylmethylether; Cyclododecylmethylether; (Ethoxymethoxy)cyclododecan; α-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
aus der Gruppe der cyclischen Ketone wie z. B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-*tert*-Pentylcyclohexanon; 5-Cyclohexadecen-1-on; 6,7-Dihydro-1,1,2,3,3-pentamethyl-4(5*H*)-indanon; 9-Cycloheptadecen-1-on; Cyclopentadecanon; Cyclohexadecanon;
aus der Gruppe der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
aus der Gruppe der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
aus der Gruppe der Ester cyclischer Alkohole wie z. B. 2-*tert*-Butylcyclohexylacetat; 4-*tert*-Butylcyclohexylacetat; 2-*tert*-Pentylcyclohexylacetat; 4-*tert*-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5- bzw. - 6-indenylacetat; 4,7-Methano-3a,4,5,6,7,7a-hexahydro-5- bzw. -6-indenylpropionat; 4,7-Methano-3a,4,5,6,7,7a-hexahydro-5- bzw. -6-indenylisobutyrat; 4,7-Methanooctahydro-5- bzw. -6-indenylacetat;
aus der Gruppe der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
aus der Gruppe der aromatischen Kohlenwasserstoffe wie z. B. Styrol und Diphenylmethan;
aus der Gruppe der araliphatischen Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
aus der Gruppe der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; α-Trichlormethylbenzylacetat; α,α-Dimethylphenylethylacetat; α,α-Dimethylphenylethylbutyrat; Cinnamylacetat; 2-Phenoxyethylisobutyrat; 4-Methoxybenzylacetat;
aus der Gruppe der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyd-diethylacetal; Hydratropaaldehyd-dimethylacetal; Phenylacetaldehyd-glycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
aus der Gruppe der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert-butylphenyl)propanal; *3-*(4*-tert-*Butylphenyl)propanal; Zimtaldehyd; α-Butylzimtaldehyd; α-Amylzimtaldehyd; α-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
aus der Gruppe der aromatischen und araliphatischen Ketone wie z. B. Acetophenon; 4-Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-*tert*-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
aus der Gruppe der aromatischen und araliphatischen Carbonsäuren und deren Ester wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; lsoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
aus der Gruppe der stickstoffhaltigen aromatischen Verbindungen wie z. B. 2,4,6-Trinitro-1,3-dimethyl-5-tert-butylbenzol; 3,5-Dinitro-2,6-dimethyl-4-tert-butylacetophenon; Zimtsäurenitril; 5-Phenyl-3-methyl-2-pentensäurenitril; 5-Phenyl-3-methylpentansäurenitril; Methylanthranilat; Methy-*N-*methylanthranilat; Schiff'sche Basen von Methylanthranilat mit 7-Hydroxy-3,7-dimethyloctanal, 2-Methyl-3-(4-tert-butylphenyl)propanal oder 2,4-Dimethyl-3-cyclohexencarbaldehyd; 6-lsopropylchinolin; 6-lsobutylchinolin; 6-sec-Butylchinolin; Indol; Skatol; 2-Methoxy-3-isopropylpyrazin; 2-Isobutyl-3-methoxypyrazin; 4-(4,8-Dimethyl-3,7-nonadienyl)-pyridin;
aus der Gruppe der Phenole, Phenylether und Phenylester wie z. B. Estragol; Anethol; Eugenol; Eugenylmethylether; Isoeugenol; lsoeugenylmethylether; Thymol; Carvacrol; Diphenylether; β-Naphthylmethylether; β-Naphthylethylether; β-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
aus der Gruppe der heterocyclischen Verbindungen wie z. B. 2,5-Dimethyl-4-hydroxy-2*H*-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
aus der Gruppe der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; *cis*- und *trans*-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1, 12-dodecandioat; Ethylen-1,13-tridecandioat; Cumarin; 2,3-Dihydrocumarin; Octahydrocumarin.

Die Verbindung der Formel (II) oder eine erfindungsgemäße Mischung (wie oben definiert) beeinflussen die sensorischen Eigenschaften von Riechstoffmischungen auf diverse Art und Weise (siehe dazu auch die untenstehende Vergleichsbeispiele).

Bevorzugt ist eine erfindungsgemäße Riechstoffmischung, vorzugsweise ein erfindungsgemäßes Parfümöl, umfassend
(a) einen, 2, 3 oder mehr weitere Holz- und/oder Ambra-Riechstoffe,
   und/oder
(b) einen, 2, 3 oder mehr Moschusriechstoffe.

Der oder die Moschusriechstoffe des Bestandteils (b) sind Riechstoffe, die einen Moschusgeruch aufweisen. Solche Riechstoffe sind dem Fachmann bekannt, da "Moschus" (musk) eine sehr wichtige Geruchsrichtung in der Parfümerie darstellt.

Erfindungsgemäß ist das Massenverhältnis der Gesamtmenge an Moschusriechstoffen, also des Bestandteils (b), zu Verbindung (II), d.h. das Gesamtgewichtsverhältnis von (b) : Verbindung (II), vorzugsweise größer oder gleich 10 : 1, bevorzugt beträgt es 10 : 1 bis 100.000 : 1, weiter bevorzugt 50 : 1 bis 100.000 : 1, besonders bevorzugt 100 : 1 bis 100.000 : 1.

Erfindungsgemäße Riechstoffmischungen, insbesondere Parfümöle, enthaltend Verbindung (II) oder eine erfindungsgemäße Mischung (wie oben definiert) und einen oder mehrere Moschusriechstoffe, insbesondere solche aus nachfolgender Tabelle 1, bei denen das Massenverhältnis der Gesamtmenge an Moschusriechstoffen (b) zu Verbindung (II) größer oder gleich 10 : ist, sind weiter bevorzugt.

Die erfindungsgemäße Verbindung (II) oder eine erfindungsgemäße Mischung (wie oben definiert) betont in solchen bevorzugten bzw. besonders bevorzugten erfindungsgemäßen Riechstoffmischungen, insbesondere solchen erfindungsgemäßen Parfümölen, sowohl den erogenen, maskulinen und animalischen Geruchscharakter (heben diesen Geruchscharakter also hervor), der allen Moschusverbindungen eigen ist und vermittelt einer erfindungsgemäßen Riechstoffmischung, insbesondere einem erfindungsgemäßen Parfümöl, dadurch einen softeren, pflegenderen (und teils kosmetischeren) und somit letztlich insgesamt hochwertigeren Geruchseindruck.

Bevorzugt ist dabei eine erfindungsgemäße Riechstoffmischung, vorzugsweise ein erfindungsgemäßes Parfümöl, wobei der oder die Moschusriechstoffe des Bestandteils (b) gewählt sind aus der Gruppe der macrocyclischen Moschusriechstoffe, der Nitro-Moschusriechstoffe (aromatische Moschusriechstoffe mit Nitrogruppe(n)), der polycyclischen Moschusriechstoffe und/oder der alicyclischen Moschusriechstoffe.

Erfindungsgemäß bevorzugte Riechstoffmischungen, vorzugsweise Parfümöle, enthalten zwei, drei oder mehr verschiedene Moschusriechstoffe als Bestandteil (b), wobei jeder der Moschusriechstoffe unabhängig von den übrigen Moschusriechstoffen vorzugsweise gewählt ist aus der Gruppe der macrocyclischen Moschusriechstoffe, der Nitro-Moschusriechstoffe (aromatische Moschusriechstoffe mit Nitrogruppe(n)), der polycyclischen Moschusriechstoffe und/oder der alicyclischen Moschusriechstoffe.

Bevorzugt werden der oder die Moschusriechstoffe im Rahmen der vorliegenden Erfindung gewählt aus nachfolgender Tabelle 1:

**Tabelle 1:**

| TYP | Produkt / Markenname | Name / CAS-Name |
|---|---|---|
| MACRO | EXALTENON | 4-Cyclopentadecen-1-one (4Z)- ; 4-Cyclopentadecen-1-on |
| MACRO | CIVETON | 9-Cycloheptadecen-1-on, (9Z)- |
| MACRO | CYCLOHEXADECANOLID, DIHYDROAMBRETTOLID | Oxacycloheptadecan-2-on, ω-Hexadecanolid |
| MACRO | ETHYLENDODECANDIOAT | 1,4-Dioxacyclohexadecane-5,16-dion |
| MACRO | GLOBALIDE® | Oxacyclohexadecen-2-on; 15-Pentadec-(11/12)-enolid |
| MACRO | ETHYLENBRASSYLAT | 1,4-Dioxacycloheptadecane-5,17-dion |
| MACRO | MUSCON | 3-Methy-cyclopentadecanon |
| MACRO | AMBRETTOLID | Oxacycloheptadec-10-en-2-on |
| MACRO | MUSCENON | 3-Methyl-cyclopentadecenon |
| MACRO | VELVIONE®, AMBRETONE | 5-Cyclohexadecen-1-on |
| MACRO | AURELIONE® | 7/8-Cyclohexadecen-1-on |
| MACRO | GLOBANONE® | 8-Cyclohexadecen-1-on |
| MACRO | lSOMUSCONE® | Cyclohexadecanon |
| MACRO | EXALTOLID, MACROLIDE® | Oxacyclohexadecan-2-on |
| MACRO | COSMONE® | 3-Methyl-(5E/Z)-cyclotetradecen-1-on |
| POLY | TRASEOLIDE® | 1-[2,3-Dihydro-1,1,2,6-tetramethyl-3-(1-methylethyl)-1H-inden-5- yl]-ethanon |
| POLY | PHANTOLIDE® | 1-(2,3-Dihydro-1,1,2,3,3,6-hexamethyl-1H-inden-5-yl)-ethanon |
| POLY | TONALIDE® | 1-(5,6,7,8-Tetrahydro-3,5,5,6,8,8-hexamethyl-2-naphthalenyl)-ethanon |
| POLY | CRYSOLIDE | 1-[6-(1,1-Dimethylethyl)-2,3-dihydro-1,1-dimethyl-1H-inden-4-yl]-ethanon |
| POLY | CHROMANOLIDE® | Tetradecansäure, 1-methylethyl ester; Cyclopenta[g]-2-benzopyran, 1,3,4,6,7,8-hexahydro-4,6,6,7,8,8-hexamethyl- |
| POLY | GALAXOLIDE® | Cyclopenta[g]-2-benzopyran, 1,3,4,6,7,8-hexahydro-4,6,6,7,8,8-hexamethyl- |
| ALICYC | HELVETOLIDE® | 1-Propanol, 2-[1-(3,3-dimethylcyclohexyl)ethoxy]-2-methyl-, 1-propanoat |
| NITRO | MOSKENE | 2,3-Dihydro-1,1,3,3,5-pentamethyl-4,6-dinitro-1H-lnden |
| NITRO | MUSK TIBETENE | 1-(1,1-Dimethylethyl)-3,4,5-trimethyl-2,6-dinitrobenzol |
| NITRO | ORINOX | 1-[4-(1,1-Dimethylethyl)-2,6-dimethylphenyl]-ethanon |
| NITRO | MUSK XYLOL | 1-(1,1-Dimethylethyl)-3,5-dimethyl-2,4,6-trinitrobenzol |
| NITRO | MUSK KETONE | 1-[4-(1,1-Dimethylethyl)-2,6-dimethyl-3,5-dinitrophenyl]-ethanon |
| NITRO | MUSK ALPHA | 1,3-Dibromo-2-methoxy-4-methyl-5-nitrobenzol |

| | | |
|---|---|---|
| MACRO = macrocyclische Moschusriechstoffe NITRO = Nitro-Mochusriechstoffe POLY = polycyclische Mochusriechstoffe ALICYC = alicyclischer Mochusriechstoff | | |

Weiter bevorzugt als Teil des Bestandteils (b) sind polycyclische und/oder macrocyclische Moschusriechstoffe, wobei sich insbesondere macrocyclische Moschusriechstoffe als besonders vorteilhaft im Sinne der Erfindung gezeigt haben, welche wiederum vorzugsweise gewählt sind aus der Gruppe bestehend aus macrocyclischen C₁₄-C₁₈-Ketonen und macrocyclischen C₁₄-C₁₈-Lactonen. Dabei wiederum sind Ketone bzw. Lactone mit einer Ringgröße von 15 bis 17 Ringatomen bevorzugt, die vorzugsweise kein, ein oder zwei Sauerstoffatome im Ring aufweisen.

Weiter bevorzugt ist eine erfindungsgemäße Riechstoffmischung, vorzugsweise ein erfindungsgemäßes Parfümöl, wobei das Massenverhältnis der Gesamtmenge an Moschusriechstoffen des Bestandteils (b) zu Verbindung (II) 10 : 1 bis 100.000 : 1, weiter bevorzugt 50 : 1 bis 100.000 : 1, besonders bevorzugt 100 : 1 bis 100.000 : 1.

Am meisten bevorzugt sind erfindungsgemäße Riechstoffmischungen, deren Bestandteil (b) umfasst oder aisgewählt ist aus: 3-Methylcyclopentadecenon (Muscenon), 15-Pentadec-(11/12)-enolid (Globalide)®, Ethylenbrassylat, Oxacyclohexadecan-2-on (Macrolide®), Cyclohexadecanon (Isomuscone®), 8-Cyclohexadecanon (Globanone®), (7/8)-Cyclohexadecanon (Aurelione®) und deren Mischungen.

In einer bevorzugten Ausführungsform wird Bestandteil (b) gewählt aus der Gruppe bestehend aus 15-Pentadec-(11/12)-enolid (Globalide®), Ethylenbrassylat und Oxacyclohexadecan-2-on (Macrolide®) und deren Mischungen.

Der animalische Charakter der Moschusriechstoffe wird durch den Zusatz der Verbindung der Formel (II) bzw. einer erfindungsgemäßen Mischung (wie oben definiert) in besonderer Weise mit den vorzugsweise ein, zwei, drei, vier, fünf, sechs, sieben, acht, neun, zehn oder mehr weiteren Riechstoffen, die vorzugsweise Bestandteil einer erfindungsgemäßen Riechstoffmischung, vorzugsweise eines erfindungsgemäß bevorzugten Parfümöls, sind, harmonisiert und abgerundet. Dadurch entsteht ein weicherer, pflegenderer und somit letztlich hochwertigerer Gesamteindruck

Wie bereits erwähnt, eignet sich die erfindungsgemäße Verbindung der Formel (II) wegen ihrer olfaktorischen Eigenschaften besonders gut für den Einsatz in Riechstoffmischungen und Parfümölen. Die Verbindung der Formel (II) kann dabei alleine oder in erfindungsgemäßen Mischungen zusammen mit der Verbindung der Formel (I) in entsprechenden Riechstoffmischungen mit einem weiteren Einzelriechstoff oder aber einer Vielzahl weiterer Riechstoffe zusammen eingesetzt werden. Besonders vorteilhaft lässt sich die Verbindung der Formel (II) mit anderen Riechstoffen, vorzugsweise ausgewählt aus den bereits oben bzw. weiter unten genannten Riechstoffen, in unterschiedlichen Mengenverhältnissen zu neuartigen Riechstoffmischungen bzw. Parfümen kombinieren.

Es wurden ferner weitere olfaktorische Effekte gefunden.

Die Erfindung betrifft ferner Verfahren zum Verstärken eines Geruchs mit einer der Noten fruchtig, blumig, würzig, holzig, moschus und/oder ambriert, umfassend den folgenden Schritt:
- Vermischen von
   einem oder mehreren Riechstoffen mit einer, mehrerer oder sämtlichen der Noten fruchtig, blumig, würzig, holzig, moschus und/oder ambriert,
   (i) mit einer Menge der erfindungsgemäßen Verbindung (II) oder einer erfindungsgemäßen Mischung (wie oben definiert), die ausreicht, den Geruchseindruck des bzw. der Riechstoffe, die eine, mehrere oder sämtliche der Noten fruchtig, blumig, würzig, holzig, moschus und/oder ambriert verursachen, zu betonen,
      oder
   (ii) mit einer erfindungsgemäßen Riechstoffmischung, vorzugsweise einem erfindungsgemäßen Parfümöl, vorzugsweise in einer der als bevorzugt gekennzeichneten Ausgestaltungen, in einer Menge, die ausreicht, den Geruchseindruck des bzw. der Riechstoffe, die eine, mehrere oder sämtliche der Noten fruchtig, blumig, würzig, holzig, moschus und/oder ambriert verursachen, zu betonen.

Die erfindungsgemäße Verbindung der Formel (II), erfindungsgemäße Mischungen bzw. erfindungsgemäße Riechstoffmischungen werden vorzugsweise zur Herstellung parfümierter Produkte (parfümierte Artikel) eingesetzt. Die sensorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Verbindung unterstreichen ihre besondere Eignung für die genannten Einsatzzwecke.

Dementsprechend wird im Rahmen der vorliegenden Erfindung ein parfümiertes Produkt angegeben, welches die Verbindung der Formel (II), eine wie oben definierte erfindungsgemäße Mischung oder eine wie oben definierte erfindungsgemäße Riechstoffmischung enthält. Hinsichtlich bevorzugter Ausgestaltungen gelten die obigen Ausführungen entsprechend.

Bevorzugte erfindungsgemäße parfümierte Produkte sind ausgewählt aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, wie z.B. Hautcremes- und - lotionen, Sonnenschutzcremes- und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und - lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Riechstoffmischungen enthaltend die Verbindung der Formel (II) oder eine wie oben definierte erfindungsgemäße Mischung können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes und -**Iotionen**, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Ein erfindungsgemäßes parfümiertes Produkt enthält Verbindung (II), eine erfindungsgemäße Mischung (wie oben definiert) oder eine Riechstoffmischung (wie oben definiert), vorzugsweise in einer sensorisch wirksamen Menge.

Für die in erfindungsgemäßen parfümierten Produkten enthaltene Verbindung der Formel (II), eine wie oben definierte erfindungsgemäße Mischung bzw. eine wie oben definierte erfindungsgemäße Riechstoffmischung und deren bevorzugte Ausführungsformen gilt das weiter oben Gesagte hier entsprechend.

Die erfindungsgemäßen zuvor genannten Riechstoffmischungen bzw. die erfindungsgemäß in den entsprechenden Produkten einzusetzenden Riechstoffmischungen können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1 ,2-Butylenglycol, Dipropylenglycol, Diethylphthalat, Triethylcitrat, lsopropylmyristat usw. Für die genannten Lösungsmittel gilt, dass diese im Rahmen des vorliegenden Textes im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Produkten enthaltene Verbindung der Formel (II), eine wie oben definierte erfindungsgemäße Mischung oder eine wie oben definierte erfindungsgemäße Riechstoffmischung können dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die in den erfindungsgemäßen parfümierten Produkten enthaltene Verbindung der Formel (II), eine wie oben definierte erfindungsgemäße Mischung oder eine wie oben definierte erfindungsgemäße Riechstoffmischung können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden.

Gegebenenfalls können die Eigenschaften der derart modifizierten Riechstoffmischungen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffmischungen kann beispielsweise durch das sogenannte Koazervationsverfahren mit Hilfe von Kapselmaterialien, z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffmischung und Cyclodextrinen oder Harnstoffderivaten in ein geeignetes Lösungsmittel, z.B. Wasser, hergestellt werden. Extrusions-Produkte können durch Verschmelzen der Riechstoffmischung mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäßen Riechstoffmischungen können demnach, wie bereits erwähnt, in konzentrierter Form, in Lösungen oder in oben beschriebener modifizierter Form für die Herstellung der entsprechenden erfindungsgemäßen parfümierten Artikel verwendet werden

Zutaten, mit denen die erfindungsgemäße Verbindung der Formel (II), eine wie oben definierte erfindungsgemäße Mischung oder eine wie oben definierte erfindungsgemäße Riechstoffmischung, vorzugsweise kombiniert werden kann, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Polyole, Tenside, Elektrolyte, organische Lösungsmittel oder Silikonderivate.

Im Folgenden werden weitere erfindungsgemäße Aspekte im Zusammenhang mit der erfindungsgemäßen Verbindung der Formel (II), einer erfindungsgemäßen Mischung (wie oben definiert) bzw. einer erfindungsgemäßen Riechstoffmischung (wie oben definiert) beschrieben.

Wie oben bereits ausgeführt, ist für tensidhaltige parfümierte Produkte die Substantivität eines Riechstoffs bzw. einer Riechstoffmischung gegenüber den bzw. deren Retention am Substrat, insbesondere Haaren oder textilen Fasern, eine weitere wichtige anwendungstechnische Anforderung.

Durch Zusatz der erfindungsgemäßen Verbindung der Formel (II) bzw. einer erfindungsgemäßen Mischung (wie oben definiert) zu einer vorgegebenen Riechstoffmischung nur geringer Substantivität und/oder Retention werden diese Eigenschaften in besonders vorteilhafter Weise verbessert. So lässt sich beispielsweise eine zwar fruchtig, blumig, und/oder würzig duftende, aber aufgrund nur mangelhafter Substantivität der enthaltenen Duftstoffe nicht zum Weitergeben eines fruchtigen, blumigen und/oder würzigen Geruchs an Wäsche (textile Fasern) oder Haare geeignete wässrige Waschlösung (bzw. ein entsprechendes Waschmittel oder Shampoo oder dergleichen) durch Zusatz der erfindungsgemäßen Verbindung der Formel (II) bzw. einer erfindungsgemäßen Mischung (wie oben definiert) in eine Lösung überführen, die einen fruchtigen, blumigen und/oder würzigen Geruch hervorragend weitergibt - und an den behandelten Substraten (Haare bzw. textile Fasern) haftet der fruchtige, blumige und/oder würzige Geruch lange an.

Die erfindungsgemäße Verbindung der Formel (II) bzw. eine erfindungsgemäße Mischung (wie oben definiert) enthaltende Riechstoffmischungen (wie oben charakterisiert) zeichnen sich durch eine hohe Substantivität (wie oben definiert) aus. Dieser Effekt zeigt sich insbesondere auf Substraten wie Haut, Haar und textilen Fasern (z.B. Wolle, Baumwolle, Leinen, synthetische Fasern).

Dieser Effekt wird weiter unten im Rahmen der anwendungstechnischen Beispiele näher erläutert.

Ein erfindungsgemäßes parfümiertes ist daher dadurch gekennzeichnet, dass das Produkt ein oder mehrere Tenside enthält.

Bevorzugt ist es, wenn das Produkt eines der folgenden ist:
ein schwach saures, alkalisches oder neutrales Reinigungsmittel, das insbesondere aus der Gruppe ausgewählt ist bestehend aus
   - Allzweckreinigern, Fußbodenreinigern, Fensterglasreinigern, Geschirrspülmittel, Bad- und Sanitärreinigern, Scheuermilch, festen und flüssigen WC-Reinigern, pulverund schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
   - Luftverbesserern in flüssiger, gelartiger oder auf einem festen Träger aufgebrachter Form oder als Aerosolspray,
   - Wachsen oder eine Polituren, die insbesondere aus der Gruppe ausgewählt ist bestehend aus Möbelpolituren, Fußbodenwachsen und Schuhcremes,
oder
   - Körperpflegemitteln, das insbesondere aus der Gruppe ausgewählt ist bestehend aus Duschgelen und Shampoos.

Die beschriebenen positiven Eigenschaften tragen dazu bei, dass die erfindungsgemäße Verbindung der Formel (II), eine erfindungsgemäße Mischung (wie oben definiert) bzw. eine erfindungsgemäße Riechstoffmischung (wie oben definiert) besonders bevorzugt in Wasch- und Reinigungsmitteln, Hygiene- oder Pflegeprodukten, insbesondere im Bereich der Körper- und Haarpflege, der Kosmetik und des Haushalts eingesetzt werden.

Besonders bevorzugte erfindungsgemäße parfümierte Produkte sind daher Wasch- und Reinigungsmittel, Hygiene- oder Pflegeprodukte, insbesondere im Bereich der Körperund Haarpflege, der Kosmetik und des Haushalts.

Eine erfindungsgemäße Riechstoffmischung wird erfindungsgemäß hergestellt, indem die erfindungsgemäße Verbindung der Formel (II) oder eine wie oben definierte erfindungsgemäße Mischung mit dem oder den weiteren Riechstoffen sowie gegebenenfalls weiteren Bestandteilen der Riechstoffmischung vermischt werden.

Gemäß einer bevorzugten Ausführungsform wird eine erfindungsgemäße Riechstoffmischung wie oben beschrieben hergestellt, wobei die erfindungsgemäße Verbindung der Formel (II) in einer Menge eingesetzt wird, die ausreicht, um in der Riechstoffmischung eine oder vorzugsweise beide der Geruchsnoten Ambra und Holz zu vermitteln, zu modifizieren und/oder zu verstärken.

Für die neben der erfindungsgemäßen Verbindung der Formel (II) bevorzugt auszuwählenden weiteren Bestandteile bzw. Riechstoffe gilt das weiter oben Gesagte entsprechend.

Wie bereits erwähnt, kann die erfindungsgemäße Verbindung der Formel (II) als Mittel zum Versehen von Haaren und/oder textilen Fasern einer oder beider Geruchsnoten Ambra und/oder Holz verwendet werden.

Dementsprechend betrifft ein weiterer Aspekt der vorliegenden Erfindung ein Verfahren zum Versehen von (a) Haar oder (b) textilen Fasern mit einer oder beiden Geruchsnoten Ambra und/oder Holz, umfassend die folgenden Schritte:
i) Bereitstellen der Verbindung der Formel (II) oder einer erfindungsgemäßen Mischung (wie oben definiert),
ii) Applizieren der Verbindung oder der erfindungsgemäßen Mischung auf (a) das Haar oder (b) die textilen Fasern.

Die Erfindung betrifft ferner ein Verfahren zum Versehen von (a) Haaren oder (b) textilen Fasern mit einer, mehrerer oder sämtlicher der Geruchsnoten fruchtig, blumig, würzig, holzig, moschus und/oder ambriert mit folgenden Schritten:
- Bereitstellen einer erfindungsgemäßen Riechstoffmischung, vorzugsweise in einer der als bevorzugt gekennzeichneten Ausgestaltungen, umfassend
   (a) Verbindung (II) oder eine erfindungsgemäße Mischung (vorzugsweise in einer der als bevorzugt gekennzeichneten Ausgestaltungen),
      und
   (b) einen oder mehrere weitere Riechstoffe, der bzw. die eine, mehrere oder sämtliche der Noten fruchtig, blumig, würzig, holzig, moschus und/oder ambriert vermitteln, wobei die Menge an Verbindung (II) ausreicht, eine oder mehrere dieser Noten zu modifizieren und/oder zu verstärken,
- Applizieren der Mischung auf (a) das Haar oder (b) die textilen Fasern.

### Beispiele

Die folgenden Beispiele erläutern die Erfindung; sofern nicht anders angegeben beziehen sich Anteile und Prozente auf das Gewicht.

Verwendete Abkürzungen:

Dipropylenglycol (DPG), Diethylphthalat (DEP), Triethylcitrat (TEC).

Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Verwendet wurde cis-Cedrandiol, das durch Epoxidation und anschließende Ringöffnung aus (-)-α-Cedren erhalten wurde. Laut NMR-Untersuchung liegt folgende Struktur für das Diol vor:

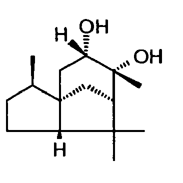

### Beispiel 1

### Umsetzung von cis-Cedrandiol mit Pentan-2-on

24 g (0,1 Mol) cis-Cedrandiol (eingesetzt wurden 26 g mit einem GC-Gehalt von 92%), 125 g (1,45 mol) Methyl-n-propyl-keton, 31,8 g (0,3 mol) Orthoameisensäuretrimethylester und 0,4 g konzentrierte Schwefelsäure wurden 24 Stunden bei 10°C gerührt. Nach dem Reaktionsende wurde Methyl-tert.-butylether zugegeben, mit einer 5%-igen Natriumhydrogencarbonat-Lösung gewaschen und die Leichtsieder abdestilliert. Nach Hinzufügen von n-Hexan kristallisierte nicht umgesetztes cis-Cedrandiol aus, das durch Filtration abgetrennt wurde. Nach Einengen des Filtrats wurden 30,4 g Rohprodukt erhalten. Gemäß GC-Analyse lag der Gehalt der Verbindungen der Formeln (I) und (II) in dem erhaltenen Produkt bei insgesamt 51 % (entsprechend 50% Ausbeute der Theorie). Durch Lösen des Rohproduktes in Dipropylenglykol (DPG) wurde hieraus eine Lösung mit einem Gehalt von 10 Gew.-% an Verbindungen der Formel (I) und (II) (mit einem Verhältnis von (I) : (II) = 4 :3) hergestellt.

Für analytische Untersuchungen wurden aus dem Rohprodukt die stereoisomeren Verbindungen der Formel (I) und (II) mittels Flüssigkeitschromatographie an Kieselgel 60 (0,04-0,063 mm) mit dem Elutionsmittel Cyclohexan/Essigsäureethylester (60/1) von einander getrennt. Die Verbindung der Formel (I) wurde mit einer Reinheit von 99,4% und die Verbindung der Formel (II) mit einer Reinheit von 96,9% erhalten.

### Verbindung der Formel (I):

1H-NMR-Daten (ppm): 4,06 d,d 9,0Hz , 6,7Hz 1H ; 2,01 d 12,1 Hz 1H ; 1,93 d 4.5Hz 1H ; 1,91 d,d,d 13,5Hz , 9,1Hz , 2,5Hz 1H ; 1,84 m 1H ; 1,80-1,70 m 3H ; 1,65- 1,37 m 7H ; 1,50 s 3H ; 1,45 s 3H ; 1,34-1,24 m 1H ; 1,16 s 3H ; 1,04 s 3H ; 0,94 t 7,4Hz 3H ; 0,82 d 7,1Hz 3H .

13C-NMR-Daten (ppm): 110,6 s ; 84,7 d ; 78,6 s ; 58,9 d ; 57,4 d ; 52,3 s ; 45,0 t ; 42,4 s ;42,0d;41,1 t ; 38,5 t ; 36,0 t ; 31,2 q ; 28,8 q ; 27,8 q ; 27,2 q ; 25,5 t ; 18,3 t ; 15,4q; 14,7 q .

Massenspektrum: 306 (1,8), 263 (30), 203 (36), 133 (31), 119 (59), 105 (30), 91 (18), 69 (40), 55 (32), 43 (100)

### Geruch: schwach Ambra

### Verbindung der Formel (II):

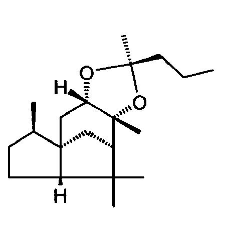

1H-NMR-Daten (ppm): 4.05 d,d 9.0Hz , 6.7Hz , 1H ; 2.00 d 12.1 Hz 1H ; 1.94 d 4.5Hz 1H ; 1.94 d,d,d 13.4Hz , 9,1 Hz , 2,5Hz 1H ; 1,88-1,50 m 8H ; 1,49 s 3H ; 1,48 s 3H ; 1,49-1,40 m 3H ; 1,34-1,24 m 1H ; 1.16 s 3H ; 1,04 s 3H ; 0,93 t 7,4Hz 3H ; 0.82 d 7,1 Hz 3H .

13C-NMR-Daten (ppm): 110,7 s ; 84,9 d ; 78,5 s ; 58,7 d ; 57,5 d ; 52,4 s ; 45,9 t ; 42,4 s ; 41,9d;41,3t;38,5t;35,8t;31,2q;28,7q;27,8q;26,7q;25,4t;18,5t;15,4q; 14,6 q .

Massenspektrum: 306 (1,2), 263 (36), 203 (46), 133 (40), 119 (68), 105 (34), 91 (19), 69 (44), 55 (32), 43 (100)

Geruch: sehr stark Ambra, Holz, erinnert an weiße Ambra, lange Haftung

### Vergleichsbeispiel

### Umsetzung von cis-Cedrandiol mit Butan-2-on

29,7 g (0,125 mol) cis-Cedrandiol (eingesetzt wurden 40 g mit einem GC-Gehalt von 74,3%), 125 g (1,74 mol) Butan-2-on, 40 g (0,375 mol) Orthoameisensäuretrimethylester und 0,5 g konzentrierte Schwefelsäure wurden 16 Stunden bei 10°C gerührt. Nach dem Reaktionsende wurde Methyl-tert.-butylether zugegeben, mit einer 5%-igen Natriumhydrogencarbonat-Lösung gewaschen und die Leichtsieder abdestilliert. Nach Hinzufügen von n-Hexan kristallisierte nicht umgesetztes cis-Cedrandiol aus, das durch Filtration abgetrennt wurde. Nach Einengen des Filtrats wurden 32 g an Rohprodukt erhalten. Gemäß GC-Analyse lag der Gehalt der Verbindungen der Formeln (Z-1) und (Z-2) in dem erhaltenen Produkt bei insgesamt 42% (entsprechend 37% Ausbeute der Theorie).

Nach Kugelrohrdestillation wurde die Verbindung der Formel (Z-1) durch Flüssigkeitschromatographie an Kieselgel 60 (0,04-0,063 mm) mit dem Elutionsmittel Cyclohexan/Methyl-tert.-butylether (50/2) und anschließender Umkristallisation aus Ethanol mit einer Reinheit von 99,8% isoliert. Die Verbindung der Formel (Z-2) wird nach Kugelrohrdestillation und Flüssigkeitschromatographie an Kieselgel 60 zusätzlich mittels Hochdruckflüssigkeitschromatographie in einer Reinheit von 99,3% erhalten.

### Verbindung der Formel (Z-1):

1H-NMR-Daten (ppm): 4,06 d,d 9,0Hz , 6,8Hz 1H ; 2,00 d 12,2 Hz 1H ; 1,94 d 4,5Hz 1H ; 1,92 d,d,d 13,5Hz , 8,9Hz , 2,6 Hz 1H ; 1,88-1,70 m 4H ; 1,66-1,40 m 5H ; 1,51 s 3H ; 1,44 s 3H ; 1,35-1,24 m 1H ; 1,16 s 3H ; 1,04 s 3H ; 1,00 t 7,6Hz 3H ; 0,81 d 7,1Hz 3H.

13C-NMR-Daten (ppm): 111,1 s ; 84,8 s ; 78,7 d ; 58,6 d ; 57,3 d ; 52,4 s ; 42,5 s ; 41,9 d ;41,1t;38,5t;35,8t;35,3t;31,2q;28,7q;27,8q;26,5q;25,4t; 15,4q;9,3q q

Massenspektrum: 292 (2,3), 263 (37), 221 (100), 203 (49), 133 (48), 119 (88), 105 (45), 69 (55), 55 (45), 43 (87), 41 (51)

### Geruch: schwach Ambra

### Verbindung der Formel (Z-2):

1H-NMR-Daten (ppm): 4,06 d,d 9,0Hz , 6,8Hz 1H ; 2,01 d 12,1Hz 1H ; 1,95 d,d,d 13,5Hz , 9,1Hz , 2,5Hz 1H ; 1,94 d 4,5Hz 1H ; 1,89-1,40 m 9H ; 1,49 s 3H ; 1,47 s 3H ; 1,34-1,25 m 1H ; 1,16 s 3H ; 1,04 s 3H ; 0,98 t 7,6Hz 3H ; 0,82 d 7,1Hz 3H .

13C-NMR-Daten (ppm): 111,1 s ; 84,9 s ; 78,6 d ; 58,7 d ; 57,4 d ; 52,4 s ; 42,5 s ; 41,9 d ; 41,3 t ; 38,5 t ; 36,1 t ; 35,8 t ; 31,2 q ; 28,7 q ; 27,8 q ; 25,9 q ; 25,4 t ; 15,4 q ; 9,5 q

Massenspektrum: 292 (1,6), 263 (37), 221 (100), 203 (53), 133 (53), 119 (96), 105 (52), 69 (61), 55 (49), 43 (96), 41 (57)

### Geruch: Ambra, Holz

In den nachfolgenden Parfümölrezepturen und Anwendungsbeispielen wurden jeweils Lösungen mit dem angegebenen Gehalt eingesetzt, wobei das Gewichtsverhältnis von der Verbindung der Formel (1) zu der Verbindung der Formel (II)) bei 4 : 3 lag.

### Erfindungsgemäßes Parfümöl-Beispiel P1:

| | Ref-1 | P1 |
|---|---|---|
| HEXENALDIMETHYLACETAL, 2,2,5-TRIMETHYL-4- | 10 | 10 |
| HEXADECEN-1,16-OLID, 7(9)Z- | 15 | 15 |
| CYCLOHEXADECENON, (8E/Z)- + CYCLOHEXADECENON, (7E)- | 140 | 140 |
| BUT-2-EN-1-ON, 1-(2,6,6-TRIMETHYL-CYCLOHEX-3-ENYL)- (E) 10%DPG | 10 | 10 |
| BRASSYLSAEUREETHANDIOLESTER | 120 | 120 |
| NONADIEN-3-OL, 3,7-DIMETHYL-1,6- | 30 | 30 |
| PYRANOL, 2-ISOBUTYL-4-METHYL-4-TETRAHYDRO-(E/Z) | 40 | 40 |
| BUTTERSAEURE-5-HEXENYLESTER, 2-METHYL- | 5 | 5 |
| ESSIGSAEUREGERANYLESTER | 40 | 40 |
| METHYL-DIHYDROJASMONAT | 400 | 400 |
| PROPIONSAEURE-2-(1-(3,3-DIMETHYLCYCLOHEXYL)-ETHOXY-2-METHYLPROPYLESTER | 30 | 30 |
| ESSIGSAEURE-3Z-HEXENYLESTER | 5 | 5 |
| SALICYLSAEURE-3Z-HEXENYLESTER | 20 | 20 |
| BICYCLO[4.4.0]DECEN, 3,4,10,10-TETRAMETHYL-3-HYDROXYETHYL-1(6) | 50 | 50 |
| CYCLOHEXANON, 3,3,5,5-TETRAMETHYL-4-(1-ETHOXYVINYL)- | 10 | 10 |
| KOHLENSAEURE-3Z-HEXENYLMETHYLESTER, 10% in DPG | 10 | 10 |
| MANDARINENOEL | 20 | 20 |
| 2(3H)-FURANON, 5-HEXYL-DIHYDRO-4-METHYL- (E/Z) | 5 | 5 |
| ESSIGSAEUREPRENYLESTER | 5 | 5 |
| TRICYCLO(5.2.1.0)DECAN, 8-FORMYL- | 15 | 15 |
| DIOXOL, 4H-4A,9-METHANOAZULENO(5,6-D)-OCTAHYDRO-2,2,5,8,8,9A-HEXAMETHYL-, 1,3-(Ambrocenide®), 0, 1 % in DPG | 20 | 0 |
| Dioxatetracyclo[6.5.1.0 ^{1,10}.0^{3,7})tetradecan, 5,7,9,9,13-Pentamethyl-5-propyl-4,6- (Verbindung der Formel (II)), 0,1 % in DPG | 0 | 20 |
| TOTAL | 1000 | 1000 |

Das Vergleichsbeispiel (Ref-1) mit Ambrocenide® riecht frisch, fruchtig und transparent, In Parfümöl P1 verleiht die Verbindung der Formel (II) der Komposition eine Eleganz und ausgeprägtere Fruchtigkeit.

### Erfindungsgemäßes Parfümöl-Beispiel P2:

| | Ref-2 | P2 |
|---|---|---|
| UNDECALACTON, GAMMA- | 2 | 2 |
| HEXADECEN-1,16-OLID, 7(9)Z- | 10 | 10 |
| CYCLOHEXADECENON, (8E/Z)- + CYCLOHEXADECENON, (7E)- | 100 | 100 |
| CARDAMOMENOEL CEYLON | 2 | 2 |
| INDAN-4-ON, 1,1,2,3,3-PENTAMETHYL-TETRAHYDRO- | 15 | 15 |
| CEDERNHOLZOEL VIRGINIA | 100 | 100 |
| DAMASCON, -BETA-, 10% in DPG | 2 | 2 |
| METHYL-DIHYDROJASMONAT | 210 | 210 |
| BICYCLO[4.4.0]DECEN, 3,4,10,10-TETRAMETHYL-3-HYDROXYETHYL-1(6) | 300 | 300 |
| IRALDEIN, GAMMA- | 60 | 60 |
| CYCLOHEXANON, 3,3,5,5-TETRAMETHYL-4-(1-ETHOXYVINYL)- | 10 | 10 |
| CYCLOPENTADECENON, 3-METHYL-5E- | 20 | 20 |
| PATCHOULI OEL | 5 | 5 |
| PENTEN-2-OL, 3,3-DIMETHYL-5-(2,2,3-TRIMETHYL-3-CYCLOPENTENYL)-4- | 20 | 20 |
| PENTANOL, 3-METHYL-5-(2,2,3-TRIMETHYL-3-CYCLOPENTENYL)-2-+ HEXANOL, 6-(2,2,3-TRIME-3-CYCLOPENTENYL)-3- | 80 | 80 |
| VANILLIN | 2 | 2 |
| ISOLONGIFOLANONETHANDIOLKETAL | 30 | 30 |
| ZIMTRINDENOEL CEYLON | 2 | 2 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 30 | 0 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 30 |
| TOTAL | 1000 | 1000 |

Das Vergleichsbeispiel (Ref-2) mit Ambrocenide® riecht blumig-würzig mit trockenen holzigen Akzenten. In Parfümöl P2 wird durch die Verbindung der Formel (II) die würzige Note unterstützt, so dass die gesamte Komposition pfeffriger und charaktervoller erscheint.

### Erfindungsgemäßes Parfümöl-Beispiel P3:

| | Ref-3 | P3 |
|---|---|---|
| HEXENALDIMETHYLACETAL, 2,2,5-TRIMETHYL-4- | 10 | 10 |
| BENZODIOXEPINON, 7-METHYL-3,4-DIHYDRO-3- | 10 | 10 |
| PROPANAL, 2-METHYL-3-(4-METHOXYPHENYL)- | 5 | 5 |
| CITRONELLOL, BETA- | 20 | 20 |
| CITRONENOEL | 15 | 15 |
| CYCLOHEXYLOXYESSIGSAEUREALLYLESTER, 10% in DPG | 10 | 10 |
| DAMASCON, TRANS-ALPHA-, 1 % in DPG | 20 | 20 |
| BUT-2-EN-1-ON, 1-(2,6,6-TRIMETHYL-CYCLOHEX-3-ENYL)- (E), 10% in DPG | 5 | 5 |
| DECALACTON GAMMA, 10% in DPG | 15 | 15 |
| BRASSYLSAEUREETHANDIOLESTER | 80 | 80 |
| NONADIEN-3-OL, 3,7-DIMETHYL-1,6- | 70 | 70 |
| PHENOL, 2-METHOXY-4-(2-PROPENYL)- | 5 | 5 |
| PYRANOL, 2-ISOBUTYL-4-METHYL-4-TETRAHYDRO- (E/Z) | 80 | 80 |
| BUTTERSAEURE-5-HEXENYLESTER, 2-METHYL- | 15 | 15 |
| METHYL-DIHYDROJASMONAT | 250 | 250 |
| PROPANAL, 2-PIPERONYL- | 30 | 30 |
| SALICYLSAEURE-3Z-HEXENYLESTER | 5 | 5 |
| INDOL, 10% in DPG | 10 | 10 |
| IRALDEIN, GAMMA- | 30 | 30 |
| BICYCLO[4.4.0]DECEN, 3,4,10,10-TETRAMETHYL-3-HYDROXYETHYL-1(6) | 80 | 80 |
| PENTADECANOLID, 1,15- | 20 | 20 |
| HEPTENAL, 2,6-DIMETHYL-5-, 10% in DPG | 3 | 3 |
| PYRIDIN, 4-DECYL-, 1% in DPG | 2 | 2 |
| CYCLOHEXEN, 2,4-DIMETHYL-1-FORMYL-3-, 10% in DPG | 10 | 10 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 5 | 0 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 5 |
| TOTAL | 805 | 805 |

Das Vergleichsbeispiel (Ref-3) mit Ambrocenide® besticht durch seine transparente Frische. In Parfümöl P3 wird durch die Verbindung der Formel (II) erreicht, dass die Komposition kosmetischer-pflegender mit mehr Volumen wahrgenommen wird.

### Erfindungsgemäßes Parfümöl-Beispiel P4:

| | Ref-4 | P4 |
|---|---|---|
| NAPHTHO[2.1-B]FURAN, DODECAHYDRO-3A.6.6.9A-TETRAMETHYL- | 5 | 5 |
| BENZOE SIAM ABS. , 50% in DPG | 10 | 10 |
| SALICYLSAEUREBENZYLESTER | 20 | 20 |
| BERGAMOTTOEL | 50 | 50 |
| CEDRYLMETHYLETHER | 40 | 40 |
| CISTUS LABDANUM | 35 | 35 |
| CISTUSOEL SPAN., 10% in DPG | 10 | 10 |
| DAMASCON, TRANS-ALPHA-, 1% in DPG | 20 | 20 |
| MYRCENOL, DIHYDRO- | 30 | 30 |
| BRASSYLSAEUREETHANDIOLESTER | 120 | 120 |
| BENZOESAEUREMETHYLESTER, 2,4-DIHYDROXY-3,6-DIMETHYL-, 1 % in DPG | 20 | 20 |
| PYRANOL, 2-ISOBUTYL-4-METHYL-4-TETRAHDRO- (E/Z) | 20 | 20 |
| PENTADECEN-1,15-OLID, (11 E/Z)- + PENTADECEN-1,15-OLID, 12E- | 30 | 30 |
| METHYL-DIHYDROJASMONAT | 300 | 300 |
| BICYCLO[4.4.0]DECEN, 3,4,10,10-TETRAMETHYL-3-HYDROXYETHYL-1(6) | 75 | 75 |
| ISOBUTYLCHINOLIN, 10% in DPG | 3 | 3 |
| CYCLOHEXANON, 3,3,5,5-TETRAMETHYL-4-(1-ETHOXYVINYL)- | 30 | 30 |
| LABDANUM ABSOLUE | 20 | 20 |
| ESSIGSAEURELINALYLESTER | 50 | 50 |
| MANDARINENOEL | 10 | 10 |
| CYCLOPENTADECENON, 3-METHYL-5E- | 10 | 10 |
| MUSKATELLER SALBEIOEL | 10 | 10 |
| OLIBANUM COEUR, 50% in TEC | 5 | 5 |
| PATCHOULIOEL | 20 | 20 |
| PYRIDIN, 4-DECYL-, 1% in DPG | 2 | 2 |
| BUTENOL, 2-ETHYL-4-(2,2,3-TRIMETHYL-3-CYCLOPENTENYL)-2E- | 5 | 5 |
| VANILLIN | 30 | 30 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 20 | 0 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 20 |
| TOTAL | 1000 | 1000 |

Das Vergleichsbeispiel (Ref-4) mit Ambrocenide® besticht durch eine schöne Holznote. In Parfümöl P4 verleiht die Verbindung der Formel (II) der Komposition mehr Volumen und die Tabaknote wird verstärkt.

### Erfindungsgemäßes Parfümöl-Beispiel P5:

| | Ref-5 | P5 |
|---|---|---|
| CYCLOHEXADECENON, (8E/Z)- + CYCLOHEXADECENON, (7E)- | 200 | 200 |
| BEIFUSSOEL | 10 | 10 |
| BERGAMOTTOEL | 60 | 60 |
| CEDRYLMETHYLETHER | 40 | 40 |
| CISTUSOEL SPAN., 10% in DPG | 10 | 10 |
| CITRONENOEL | 20 | 20 |
| CUMARIN | 40 | 40 |
| CYCLOHEXYLOXYESSIGSAEUREALLYLESTER, 10% in DPG | 5 | 5 |
| MYRCENOL, DIHYDRO- | 50 | 50 |
| BRASSYLSAEUREETHANDIOLESTER | 120 | 120 |
| BENZOESAEUREMETHYLESTER, 2,4-DIHYDROXY-3,6-DIMETHYL-, 10% in DPG | 20 | 20 |
| METHYL-DIHYDROJASMONAT | 30 | 30 |
| IRALDEIN, GAMMA- | 20 | 20 |
| BICYCLO[4.4.0]DECEN, 3,4,10,10-TETRAMETHYL-3-HYDROXYETHYL-1(6) | 120 | 120 |
| KRAUSEMINZOEL, 10% in DPG | 10 | 10 |
| LAVENDELOEL | 10 | 10 |
| LINALOOL | 20 | 20 |
| ESSIGSAEURELINALYLESTER | 20 | 20 |
| INDENO[1.2-D]-M-DIOXIN, 2,4-DIMETHYL-TETRAHYDRO- | 90 | 90 |
| MUSKATELLER SALBEI ABSOLUE | 5 | 5 |
| NELKENBLUETENOEL | 10 | 10 |
| VANILLIN | 20 | 20 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG | 20 | 0 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 20 |
| TOTAL | 950 | 950 |

Das Vergleichsbeispiel (Ref-5) mit Ambrocenide® wirkt sehr strahlend und intensiv lavendelartig. In Parfümöl P5 wird durch die Verbindung der Formel (II) die gesamte Komposition natürlicher lavendelartig, und erscheint dadurch hochwertiger mit mehr Volumen.

### Erfindungsgemäßes Parfümöl-Beispiel P6:

| | Ref-6 | P6 |
|---|---|---|
| HEXADECEN-1,16-OLID, 7(9)Z- | 20 | 20 |
| CYCLOHEXADECENON, (8E/Z)- + CYCLOHEXADECENON, (7E)- | 200 | 200 |
| INDAN-4-ON, 1,1,2,3,3-PENTAMETHYL-TETRAHYDRO- | 20 | 20 |
| BRASSYLSAEUREETHANDIOLESTER | 150 | 150 |
| BUTTERSAEURE-5-HEXENYLESTER, 2-METHYL- | 5 | 5 |
| PENTADECEN-1,15-OLID, (11 E/Z)- + PENTADECEN-1,15-OLID, 12E- | 90 | 90 |
| METHYL-DIHYDROJASMONAT | 210 | 210 |
| BICYCLO[4.4.0]DECEN, 3,4,10,10-TETRAMETHYL-3-HYDROXYETHYL-1(6) | 200 | 200 |
| CYCLOHEXANON, 3,3,5,5-TETRAMETHYL-4-(1-ETHOXYVINYL)- | 20 | 20 |
| PENTEN-2-OL, 3,3-DIMETHYL-5-(2,2,3-TRIMETHYL-3-CYCLOPENTENYL)-4- | 5 | 5 |
| ISOLONGIFOLANONETHANDIOLKETAL | 70 | 70 |
| DIOXOL, 4H-4A,9-METHANOAZULENO(5,6-D)-OCTAHYDRO-2,2,5,8,8,9A-HEXAMETHYL-, 1,3- (Ambrocenide®), 0,1% in DPG 5 | 5 | 5 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 0 |
| TOTAL | 995 | 995 |

Das Vergleichsbeispiel (Ref-6) mit Ambrocenide® besitzt eine kühle Transparenz gepaart mit einer trockenen Holzigkeit. In Parfümöl P6 verleiht die Verbindung der Formel (II) der gesamten Komposition eine ausgeprägte Harmonie und Wärme, wobei die Fruchtigkeit verstärkt (gepusht) wird hin zu einer schönen Pflaumennote gepaart mit Tabakeffekten.

### Erfindungsgemäßes Parfümöl-Beispiel P7:

| | Ref-7 | P7 |
|---|---|---|
| NAPHTHO[2.1-B]FURAN, DODECAHYDRO-3A.6.6.9A-TETRAMETHYL- | 5 | 5 |
| SALICYLSAEUREBENZYLESTER | 50 | 50 |
| BERGAMOTTOEL | 50 | 50 |
| CUMARIN | 5 | 5 |
| DAMASCON, TRANS-ALPHA-, 1% in DPG | 20 | 20 |
| DECALACTON GAMMA, 10% in DPG | 15 | 15 |
| BRASSYLSAEUREETHANDIOLESTER | 30 | 30 |
| NONADIEN-3-OL, 3,7-DIMETHYL-1,6- | 60 | 60 |
| ESSIGSAEURE-3,7-DIME-1,6-NONADIEN-3-YLESTER | 50 | 50 |
| PYRANOL, 2-ISOBUTYL-4-METHYL-4-TETRAHYDRO- (E/Z) | 70 | 70 |
| BUTTERSAEURE-5-HEXENYLESTER, 2-METHYL- | 5 | 5 |
| BICYCLO[2.2.2]OCTEN, 6-ME-8-ISOPROPYL-2(3)-(1,3-DIOXOLAN-2-YL | 5 | 5 |
| METHYL-DIHYDROJASMONAT | 280 | 280 |
| PROPANAL, 2-PIPERONYL- | 20 | 20 |
| BENZALDEHYD, 3,4-METHYLENDIOXY- | 10 | 10 |
| PROPIONSAEURE-2-(1-(3,3-DIMETHYLCYCLOHEXYL)-ETHOXY-2-METHYLPROPYLESTER | 60 | 60 |
| SALICYLSAEURE-3Z-HEXENYLESTER | 5 | 5 |
| BICYCLO[4.4.0]DECEN, 3,4,10,10-TETRAME-3-HYDROXYETHYL-1(6) | 120 | 120 |
| PENTADECANOLID, 1,15- | 30 | 30 |
| MANDARINENOEL | 20 | 20 |
| CYCLOPENTADECENON, 3-METHYL-5E- | 3 | 3 |
| BUTANOL, 2-METHYL-4-PHENYL-2- | 10 | 10 |
| PYRIDIN, 4-DECYL-, 1% in DPG | 2 | 2 |
| PENTEN-2-OL, 3,3-DIMETHYL-5-(2,2,3-TRIMETHYL-3-CYCLOPENTENYL)-4- | 20 | 20 |
| CYCLOHEXANOL, 4-(3-METHYL-BUTYL)- (E/Z) | 15 | 15 |
| VANILLIN | 20 | 20 |
| DIOXOL, 4H-4A,9-METHANOAZULENO(5,6-D)-OCTAHYDRO-2,2,5,8,8,9A-HEXAMETHYL-, 1,3-(Ambrocenide®), 0,1% in DPG | 5 | 0 |
| Dioxatetracyclo[6.5.1.0^{1,10}.0^{3,7})tetradecan, 5,7,9,9,13-Pentamethyl-5-propyl-4,6- (Verbindung der Formel (II)), 0,1% in DPG | 0 | 5 |
| DIPROPYLENGLYKOL | 15 | 15 |
| TOTAL | 1000 | 1000 |

Durch den Austausch von Ambrocenide® des Vergleichsbeispiel (Ref-7) durch die Verbindung der Formel (II) wirkt die gesamte Komposition P7 voluminöser, eleganter und somit auch hochwertiger, Parfümöl P7 wirkt komplexer und die Coumarin Note wird sehr schön harmonisiert.

### Erfindungsgemäßes Parfümöl-Beispiel P8:

| | Ref-8 | P8 |
|---|---|---|
| Ethylacetoacetat | 60 | 60 |
| Propanal, 2-Methyl-2-(4-ethylbenzyl)- | 25 | 25 |
| Cyclohexen, 2,4-Dimethyl-1-formyl-3- (E/Z) | 15 | 15 |
| Hexenol Cis-3 | 130 | 130 |
| Kohlensäure-3Z-hexenylmethylester | 20 | 20 |
| Cyclohexyloxyessigsäureallylester | 25 | 25 |
| Benzenmethanol, .alpha.-methyl-, 1-acetat | 40 | 40 |
| Linalylacetat | 80 | 80 |
| Myrcenol, Dihydro- | 650 | 650 |
| Mandarinenöl | 260 | 260 |
| Grapefruitöl | 260 | 260 |
| Hexenaldimethylacetal, 2,2,5-Trimethyl-4- | 170 | 170 |
| Methylanthranilat, 10% DPG | 155 | 155 |
| CardamomÖl | 5 | 5 |
| Red Berry Extract | 25 | 25 |
| Muskatnussöl | 20 | 20 |
| Ethylbutyrat, 10% DPG | 10 | 10 |
| Undecalacton, gamma- | 65 | 65 |
| Pentansäureethylester, 2-Methyl-, 10% DPG | 15 | 15 |
| Heptansaure, 2-propen-1-yl ester | 40 | 40 |
| Ethylmaltol | 15 | 15 |
| Benzenepropanal, alpha-Methyl-4-(1.1-dimethylethyl)- | 250 | 250 |
| 1.3-Benzodioxol-5-propanal, alpha-Methyl- | 200 | 200 |
| Hydroxycitronellal | 200 | 200 |
| 4-Methyl-2-(2-methylpropyl)oxan-4-ol | 260 | 260 |
| 1.6-Octadien-3-ol, 3.7-Dimethyl- | 40 | 40 |
| Nonadien-3-ol, 3,7-Dimethyl-1,6- | 130 | 130 |
| Buttersäure,1,1-dimethyh-2-phenylethylester | 65 | 65 |
| Phenylethylalkohol | 40 | 40 |
| Propionsäure-2-phenoxyethylester, 2-Methyl- | 40 | 40 |
| 2-Buten-1-on, 1-(2.6.6-Trimethyl-2-cyclohexen-1-yl)- (E) | 40 | 40 |
| 2-Buten-1-on, 1-(2.6.6-Trimethyl-1.3-cyclohexadien-1-yl)- | 5 | 5 |
| Methyl-dihydrojasmonat | 390 | 390 |
| 3-Buten-2-on, 3-Methyl-4-(2.6.6-trimethyl-2-cyclohexen-1-yl)- | 220 | 220 |
| Vanillin | 230 | 230 |
| Cinnamon Bark Oil | 15 | 15 |
| 2H-1-Benzopyran-2-on | 200 | 200 |
| Cyclohexanolacetat, 4-(1.1-Dimethylethyl)- (Z) | 50 | 50 |
| Cedarwood Oil | 15 | 15 |
| Bicyclo[4.4.0]decen, 3,4,10,10-Tetramethyl-3-hydroxyethyl-1 (6) | 1.500 | 1.500 |
| Cyclododecan, (Ethoxymethoxy)- | 300 | 300 |
| 2-Buten-1-ol, 2-Ethyl-4-(2.2.3-trimethyl-3-cyclopenten-1-yl)- (2E) | 130 | 130 |
| Dioxol, 4H-4a,9-Methanoazuleno(5,6-d)-octahydro-2,2,5,8,8,9a-hexamethyl-, 1,3- (Ambrocenide®), 10,0% in DPG | 30 | |
| Dioxatetracyclo[6.5.1.0^{1,10}.0^{3,7})tetradecan, 5,7,9,9,13-Pentamethyl-5-propyl-4,6- (Verbindung der Formel (II)), 10,0% in DPG | | 30 |
| 5H-3.5a-Epoxynaphth[2.1-c]oxepin, Dodecahydro-3.8.8.11a-tetramethyl- | 1.200 | 1.200 |
| Naphtho[2.1-b]furan, Dodecahydro-3a.6.6.9a-tetramethyl- | 220 | 220 |
| Dipropylenglykol | 175 | 175 |
| TOTAL | 8.030 | 8.030 |

Das Vergleichsbeispiel Ref-8 mit Ambrocenide® zeichnet sich durch den Widerspruch zwischen frischen holzigen Noten und süßen balsamischen Aspekten aus. Der Austausch von Ambrocenide® durch die Verbindung der Formel (II) verleiht der gesamten Komposition mehr Impact, Volumen, Charakter gepaart mit samtigen-pudrigen Aspekten.

### Erfindungsgemäßes Parfümöl-Beispiel P9:

| | Ref-9 | P9 |
|---|---|---|
| Undecenal, 2,6,10-Trimethyl-9- | 15,00 | 15,00 |
| Propanal, 2-Methyl-2-(4-ethylbenzyl)- | 3,00 | 3,00 |
| Cis-3 Hexenylacetat | 3,00 | 3,00 |
| Cyclohexen, 2,4-Dimethyl-1-formyl-3- (E/Z) | 2,00 | 2,00 |
| Tricyclo(5.2.1.0)decan, 8-Formyl- | 2,00 | 2,00 |
| Cyclohexyloxyessigsäureallylester | 4,00 | 4,00 |
| Heptenal, 2,6-Dimethyl-5- | 2,00 | 2,00 |
| Identoil® Bergamot | 45,00 | 45,00 |
| Myrcenol, Dihydro- | 90,00 | 90,00 |
| 2.6-Octadienal, 3.7-Dimethyl- (2E/Z) | 10,00 | 10,00 |
| 2-Pentennitril, 3-Methyl-5-phenyl- (2E/Z) | 3,00 | 3,00 |
| Orangenöl | 30,00 | 30,00 |
| Hexenaldimethylacetal, 2,2,5-Trimethyl-4- | 2,00 | 2,00 |
| Lavandinöl | 10,00 | 10,00 |
| Cardamomöl | 1,00 | 1,00 |
| Pentansäureethylester, 2-Methyl- , 10% DPG | 10,00 | 10,00 |
| Benzenepropanal, alpha-Methyl-4-(1.1-dimethylethyl)- | 40,00 | 40,00 |
| 2H-1.5-Benzodioxepin-3(4H)-on, 7-Methyl- | 20,00 | 20,00 |
| Benzenepropanol, beta.beta.3-Trimethyl- | 40,00 | 40,00 |
| Geraniumöl | 2,00 | 2,00 |
| 3-Buten-2-on, 1-(2.4.4-Trimethyl-2-cyclohexen-1-yl)- (2E) | 10,00 | 10,00 |
| alpha-Hexylcinnamaldehyd | 60,00 | 60,00 |
| Cyclopentanessigsäuremethylester, 3-Oxo-2-pentyl- (E) | 90,00 | 90,00 |
| Propannitril, 3-(3-Hexenyloxy)- (Z) | 2,00 | 2,00 |
| 3-Buten-2-on, 4-(2.6.6-Trimethyl-1-cyclohexen-1-yl)- | 5,00 | 5,00 |
| Benzol, 1-Methoxy-4-(1-propenyl)- (E) | 10,00 | 10,00 |
| Ethanon, 1-(2-Benzofuranyl)- | 2,00 | 2,00 |
| Cyclohexanolacetat, 4-(1.1-Dimethylethyl)- (Z) | 30,00 | 30,00 |
| Cedarwood Oil (Zedernholzöl) | 15,00 | 15,00 |
| Cyclohexanpropanol, alpha-Ethyl-2.2.6-trimethyl- (E/Z) | 30,00 | 30,00 |
| Bicyclo[4.4.0]decen, 3,4,10,10-Tetramethyl-3-hydroxyethyl-1(6) | 100,00 | 100,00 |
| Cyclododecan, (Ethoxymethoxy)- | 30,00 | 30,00 |
| Patchouliöl | 10,00 | 10,00 |
| 2-Buten-1-ol, 2-Ethyl-4-(2.2.3-trimethyl-3-cyclopenten-1-yl)- (2E) | 30,00 | 30,00 |
| Benzoesäuremethylester, 2,4-Dihydroxy-3,6-dimethyl- | 3,00 | 3,00 |
| Tetramethyl dodecahydro-3a,6,6,9a-naphtho(2,1-b)furan | 12,00 | 12,00 |
| Isolongifolanonethandiolketal | 50,00 | 50,00 |
| Dioxol, 4H-4a,9-Methanoazuleno(5,6-d)-octahydro-2,2,5,8,8,9a-hexamethyl-, 1,3- (Ambrocenide®), 10,0% in DPG | 2,00 | |
| Dioxatetracyclo[6.5.1.0^{1,10}.0^{3,7})tetradecan, 5,7,9,9,13-Pentamethyl-5-propyl-4,6- (Verbindung der Formel (II)), 10,0% in DPG | | 2,00 |
| Pentadecanolid, 1,15- | 30,00 | 30,00 |
| Cyclohexadecenon, 8E/Z- | 120,00 | 120,00 |
| Dipropylenglykol | 25,00 | 25,00 |
| TOTAL | 1.000,00 | 1.000,00 |

Das Vergleichsbeispiel Ref-9 mit Ambrocenide® ist ein moderner Fougere Typ mit einer wässrigen Citrus Kopfnote und einem holzigen Fond. Durch den Austausch von Ambrocenide® gegen die Verbindung der Formel (II) wird der weiße Holz, sowie Zedern- und Sandelholzaspekt verstärkt.

### Erfindungsgemäßes Parfümöl-Beispiele P10:

| | Ref-10 | P10 |
|---|---|---|
| Undecenal, 2,6,10-Trimethyl-9- | 8 | 8 |
| 10-Undecenal | 8 | 8 |
| Cyclohexen, 2,4-Dimethyl-1-formyl-3- (E/Z) | 5 | 5 |
| Kohlensäure-3Z-hexenylmethylester | 8 | 8 |
| Indeno[1.2-d]-1.3-dioxin, 4.4a.5.9b-Tetrahydro-2.4-Dimethyl- | 70 | 70 |
| Cyclohexanolacetat, 3.3.5-Trimethyl- | 20 | 20 |
| Orangenöl | 20 | 20 |
| Methyl Naphthyl Ketone Beta | 5 | 5 |
| Ethanon, 1-(3-Methyl-2-benzofuranyl)- | 2 | 2 |
| 2-Butanon, 4-(4-Hydroxyphenyl)- | 1 | 1 |
| Peach Base | 8 | 8 |
| Pyranol, 2-Isobutyl-4-methyl-4-tetrahydro- (E/Z) | 10 | 10 |
| 1.6-Octadien-3-ol, 3.7-Dimethyl- | 90 | 90 |
| 2H-Pyran, Tetrahydro-2-methyl-2-(2-methyl-1-propenyl)- (E/Z) | 3 | 3 |
| 2-Phenylethanol | 70 | 70 |
| Benzenpentanol, beta-Methyl- | 25 | 25 |
| 2.6-Octadien-1-olacetat, 3.7-Dimethyl- (2E/Z) | 30 | 30 |
| But-2-en-1-on, 1-(2,6,6-Trimethyl-cyclohex-3-enyl)- (E) | 3 | 3 |
| Benzoesäurepropylester | 10 | 10 |
| Methyl-dihydrojasmonat (E) | 70 | 70 |
| Amyl Salicylat N/Iso | 17 | 17 |
| Salicylsäurebenzylester | 80 | 80 |
| Cis-3 Hexenylsalicylat | 10 | 10 |
| Benzoesäurehexylester, 2-Hydroxy- | 33 | 33 |
| 3-Decen-5-ol, 4-Methyl- | 5 | 5 |
| Orris Base | 5 | 5 |
| 3-Buten-2-on, 3-Methyl-4-(2.6.6-trimethyl-2-cyclohexen-1-yl)- | 80 | 80 |
| Benzaldehyd, 3,4-Methylendioxy- | 10 | 10 |
| Vanillin | 4 | 4 |
| Zimtalkohol | 4 | 4 |
| 2H-1-Benzopyran-2-on | 8 | 8 |
| Acetylcedren | 70 | 70 |
| Cyclododecan, (Ethoxymethoxy)- | 40 | 40 |
| 2-Pentanon, 4-Cyclohexyl-4-methyl- | 8 | 8 |
| Patchouli Base | 37 | 37 |
| Patchouliöl | 33 | 33 |
| Dioxol, 4H-4a,9-Methanoazuleno(5,6-d)-octahydro-2,2,5,8,8,9a-hexamethyl-, 1,3- (Ambrocenide®), 1,0% in DPG | 20 | |
| Dioxatetracyclo[6.5.1.0^{1,10}.0^{3,7})tetradecan, 5,7,9,9,13-Pentamethyl-5-propyl-4,6- (Verbindung der Formel (II) 10,0% in DPG | | 2 |
| Pentadecen-1,15-olid, (11E/Z)- + Pentadecen-1,15-olid, 12E- | 40 | 40 |
| Triethylcitrat | 30 | 48 |
| TOTAL | 1000 | 1000 |

Das Vergleichsbeispiel Ref-10 mit Ambrocenide® ist ein floraler Chypre Typ mit einer aldehydigen Kopfnote, gepaart mit floralen Herznoten und abgerundet durch Patchouli und Moschus. Der Austausch von Ambrocenide® gegen die Verbindung der Formel (II) verleiht der gesamten Komposition einen pflegenderen, wärmeren und voluminöseren Charakter, zusätzlich wird die Haftung verbessert.

Die Parfümöle P1, P2, P3, P4, P5, P6, P7, P8, P9 und P10 aus den obigen Parfümöl-Beispielen wurden jeweils separat in die nachfolgenden Formulierungen eingearbeitet.

Die oben bei dem jeweiligen Parfümöl beschriebenen geruchlichen Effekte wurden jeweils auch in den nachfolgenden Formulierungen beobachtet.

### Formulierungsbeispiele

### Beispiel F1 - Waschpulver

| **Material** | **Chemischer Name** | **Funktion** | **Gew.-%** | **Gew.- %** |
|---|---|---|---|---|
| Natrium Metasilicat Pentahydrat | Sodium Metasilicate Pentahydrate | | Ad 100 | Ad 100 |
| Natrium hydrogen-carbonat | Sodium hydrogen carbonate | Alkali | 15,0 | 15,0 |
| Natrium-percarbonat | Sodium carbonate peroxyhydrate | Bleichmittel | 15,0 | 15,0 |
| Peractive AC Blue | TAED / Na-Carboxymethylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl P1, P2, P3, P4, P5, P6, P7, P8, P9 bzw. P10 | | Parfum (Fragrance) | 0,30 | 0,50 |

### Beispiel F2 - Allzweckreiniger

| **Material** | **Chemischer Name** | **Funktion** | **Gew.-%** | **Gew.₋ %** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl P1, P2, P3, P4, P 5, P 6, P 7, P8, P9 bzw. P10 | | Parfum (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butyl-paraben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl P1, P2, P3, P4, P5, P6, P7, P8, P9 bzw. P10 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butyl-paraben, Propylparaben, Isobutylparaben | 0,5 | 0,5 |
| Natriumchlorid | Sodium Chloride | 1,4 | 1,4 |
| Citronensäure Monohydrat kristallin | CitricAcid | 1,3 | 1,3 |
| Parfumöl P1, P2, P3, P4, P5, P6, P7, P8, P9 bzw. P10 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammonium-ethosulfate | Kationisches Tensid | 16,6 | 16,6 |
| Mergal K9N | 5-Chloro-2-methyl-3-(2H)-isothiazolone und 2-methyl-3-(2H)-isothiazolone | Konservierungsmittel | 0,10 | 0,10 |
| Dow Corning 1520 Antifoam | Polydimethyl-siloxane | Entschäumer | 0,30 | 0,30 |
| Magnesium Chlorid 1%ige Lösung | Magnesium Chlorid Lösung | Konsistenzgeber | 10,00 | 10,00 |
| Parfumöl P1, P2, P3, P4, P5, P6, P7, P8, P9 bzw. P10 | | Fragrance | 0,55 | 0,75 |

### Beispiel F6 - Eau de Coloqne/Eau de Toilette

| **Inhaltsstoffe** | **Gew.%** | **Gew.%** |
|---|---|---|
| Parfumöl P1, P2, P3, P4, P5, P6, P7, P8, P9 bzw. P10 | 5 | 10 |
| Ethanol | Ad 100 | Ad 100 |
| Wasser | 18 | 10 |

### Beispiel F7 - Aerosol-Pumpspray

| **Inhaltsstoffe** | **Gew.%** | **Gew.%** |
|---|---|---|
| Parfumöl P1, P2, P3, P4, P5, P6, P7, P8, P9 bzw. P10 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.%** | **Gew.%** | **Gew.%** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfumöl P1, P2, P3, P4, P5, P6, P7, P8, P9 bzw. P10 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest 2066([[(Phosphonomethyl)imino]bis[(ethylennitrilo)bis (methylen)]]tetrakis-phosphonsäure, Natriumsalz) | 2,8 | 2,8 | 2,8 |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl P1, P2, P3, P4, P5, P6, P7, P8, P9 bzw. P10 | 0,25 | 0,35 | 0,5 |
| EDTA | 1,0 | 1,0 | 1,0 |

### Beispiel F10 - Flüssigwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl P1, P2, P3, P4, P5, P6, P7, P8, P9 bzw. P10 | 0,5 |

### Beispiel F11 - Flüssigwaschmittel Konzentrat

| **Inhaltsstoffe** | **Gew.-%** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl P1, P2, P3, P4, P5, P6, P7, P8, P9 bzw. P10 | 0,7 |

### Anwendungstechnische Beispiele:

### Beispiel Eigenhaftung: Verbindung der Formel (II) versus Ambrocenide®:

Der jeweils zu untersuchende Riechstoff wird als 10%ige Lösung in DPG auf codierte Riechstreifen getaucht, d.h. der zu untersuchende Riechstoff wird durch Eintauchen des jeweiligen Riechstreifens in die jeweilige Lösung des zu untersuchenden Riechstoffs auf den Riechstreifen aufgebracht, und unmittelbar anschließend in folgenden zeitlichen Abständen geruchlich bewertet:
1 Stunde; 3 Stunden; 10 Stunden; 1 Tag; 3 Tage; 10 Tage; 1 Monat; 3 Monate. Die Probanden bewerten die Geruchsintensität auf einer Skala von 1 = geruchlos bis 9 = sehr stark.

Getestet wird solange, bis der Intensitäts-Mittelwert der Prüfer unter den Wert 1,5 (sehr schwach) fällt. Vergeben 50 % der Probanden eine Intensität von 1, und machen damit deutlich, dass der auf dem Riechstreifen getauchte Riechstoff nicht mehr wahrgenommen wird, wird der Test für beendet erklärt. Längstenfalls wird für 3 Monate getestet.

| | Verbindung der Formel (II), 10% in DPG | Ambrocenide®, 10 % in DPG |
|---|---|---|
| Eigenhaftung | mehr als 3 Monate | 10 Tage |

Die Verbindung der Formel (II) zeigt eine deutlich längere Haftung am Riechstreifen als Ambrocenide®.

### Beispiel Substantivität auf Haar: Verbindung der Formel (II) versus Ambrocenide®:

Der zu untersuchende Riechstoff wird in einer Menge von 0,6 Gew.-% als 50%ige Lösung in DPG in eine Shampoo-Formulierung analog zu Beispiel F8 anstelle des dort eingesetzten Parfümöls eingearbeitet. Für jeden zu untersuchenden Riechstoff werden 2 Haarsträhnen benötigt. Außerdem wird als Referenz jeweils ein Haarsträhnenpärchen mit der unparfümierten Shampoo-Formulierung von Beispiel F8 gewaschen.

Alle Haarsträhnen werden zusammen in einem 2 1 Becherglas mit Neutralshampoo gewaschen (mind. 2 h einweichen). Dann werden die Strähnen unter fließendem Wasser gut ausgespült und dann bei Raumtemperatur (ca. 23°C) getrocknet. Es werden von jedem Shampoo 100 ml einer 20 %igen wässrigen Lösung hergestellt (ebenso unparfümierte Masse). Nun wird je Shampoo ein Strähnenpaar gemeinsam für 2 min. in der Lösung gewaschen. Anschließend werden diese zwei Strähnen gemeinsam 20 sec. unter fließendem, handwarmem Wasser gespült. Beide Haarsträhnen werden gekämmt. Eine Haarsträhne wird nass in Alufolie eingewickelt. Die zweite Haarsträhne wird mit einem Haartrockner (Fön) getrocknet.

Ein Probandenpanel bewertet die vorbereiteten Haarsträhnen durch Riechen. Die Probanden bewerten die Intensität der Proben auf einer Skala von 0 (geruchlos) bis 6 (sehr stark).

### Reihenfolge:

### a) frisch gewaschenes, trockenes Haar

### b) frisch gewaschenes Haar, nass

| Substantivität auf Haar | Verbindung der Formel (II) | Ambrocenide® |
|---|---|---|
| Nasses Haar | 1,4 | 1,2 |
| Trockenes Haar | 1,7 | 0,5 |

Der Intensitätswert der Verbindung der Formel (II) ist auf trockenem Haar um mehr als den Faktor 3 höher verglichen mit Ambrocenide®.

### Beispiel Substantivität auf Baumwolle: Verbindung der Formel (II) versus Ambrocenide®:

Der zu untersuchende Riechstoff wird in einer Menge von 0,5 Gew.-% als 50%ige Lösung in DPG in eine Weichspüler-Formulierung analog zu Beispiel F5 anstelle des dort eingesetzten Parfümöls eingearbeitet.

Baumwolllappen werden bei 95 °C in einer Waschmaschine neutral gewaschen und bei Raumtemperatur offen getrocknet.

Jeweils zwei gleich codierte Baumwolllappen werden mit 370 g einer 1 %igen wässrigen Weichspülerlauge enthaltend den jeweils zu untersuchenden Riechstoff in einem Topf der Linitest - Maschine im Weichspülprogramm gespült. Anschließend wird die Lauge der einzelnen Töpfe abgegossen, die Lappen pro Topf ausgewrungen und alle Lappenpaare als Paar 20 sec. ausgeschleudert. Dann wird von jedem Lappenpaar ein Lappen nass eingeschweißt, und einer zum Trocknen aufgehängt.

Ein Probandenpanel bewertet die vorbereiteten Proben durch Riechen.

Die Probanden bewerten die Intensität der Proben auf einer Skala von 0 (geruchlos) bis 6 (sehr stark)

Begonnen wird jeweils mit dem unparfümierten Muster und dann die Proben.

### Reihenfolge:

### a) trockene Wäsche

### b) nasse Wäsche

| Substantivität auf Baumwolle | Verbindung der Formel (II) | Ambrocenide® |
|---|---|---|
| Nasse Wäsche | 1,6 | 1,2 |
| Trockene Wäsche | 1,5 | 0,8 |

Der Intensitätswert der Verbindung der Formel (II) ist auf trockener Wäsche um den Faktor 2 höher verglichen mit Ambrocenide®, und auch die Intensität von nasser Wäsche wird für die Verbindung der Formel (II) höher bewertet.

Darüber hinaus wurden die trockenen Baumwolllappen über einen längeren Zeitraum gelagert und jeweils nach mehreren Tagen wie oben beschrieben bewertet.

| | | |
|---|---|---|
| Tag 3 | Verbindung der Formel (II) | 2,00 |
| | Am brocenide® | 1,20 |
| Tag 8 | Verbindung der Formel (II) | 1,79 |
| | Ambrocenide® | 0,93 |
| Tag 10 | Verbindung der Formel (II) | 1,80 |
| | Ambrocenide® | 1,10 |
| Tag 14 | Verbindung der Formel (II) | 1,69 |
| | Ambrocenide® | 0,92 |
| Tag 18 | Verbindung der Formel (II) | 1,80 |
| | Ambrocenide® | 0,50 |

Die Intensität der Verbindung der Formel (II) wird auch nach einem Zeitraum von 18 Tagen immer noch als deutlich stärker beurteilt verglichen mit Ambrocenide®.

### Beispiel Ausgiebigkeit: Verbindung der Formel (I) versus Verbindung der Formel (II)

Der zu prüfende Riechstoff wurde jeweils als 10%ige Lösung in DPG (entsprechend Stufe 1) in Verdünnungsschritten mit Diethylphthalat (DEP) verdünnt, so dass weiter verdünnte Lösungen des jeweils zu prüfenden Riechstoffs der Verdünnungsstufen 2 bis 7 erhalten wurden.

| Stufe 1 | Stufe 2 | Stufe 3 | Stufe 4 | Stufe 5 | Stufe 6 | Stufe 7 |
|---|---|---|---|---|---|---|
| 10% in DPG | 1 % in DPG und DEP | 0,1% in DPG und DEP | 0,01% in DPG und DEP | 0,001% in DPG und DEP | 0,0001% in DPG und DEP | 0,00001% in DPG und DEP |

Die einzelnen Lösungen werden auf Riechstreifen getaucht und von einem Probandenpanel durch Riechen beurteilt. Die Probanden riechen und bewerten von der niedrigsten gereichten Konzentration an, d.h. beginnend mit der Verdünnung der Stufe 7.

Von den Probanden wird diejenige Verdünnungsstufe markiert, in der ein sensorischer Unterschied wahrnehmbar ist.

| | Verbindung der Formel (I) | Verbindung der Formel (II) |
|---|---|---|
| Ausgiebigkeit | 2 | 4 |

Die Verbindung der Formel (II) ist im Vergleich zu der Verbindung der Formel (I) noch in einer um den Faktor 100 niedrigeren Konzentration wahrnehmbar.

### Beispiel Intensität Verbindung der Formel (I) versus Verbindung der Formel (II)

Die jeweils zu untersuchende Probe wird als 10%ige Lösung in DPG auf einen Riechstreifen getaucht. Ein Probandenpanel bewertet die Probe durch Riechen. Die Probanden bewerten die Intensität der Verbindungen auf einer Skala von 1 (geruchlos) bis 9 (sehr stark).

| | Verbindung der Formel (I), 10% in DPG | Verbindung der Formel (II), 10% in DPG |
|---|---|---|
| Intensität | 2 | 6 |

Die Verbindung der Formel (1) ist nur sehr schwach wahrnehmbar, wohingegen die Verbindung der Formel (II) deutlich stärker wahrgenommen wird.

### Beispiel Intensität in Luft: Verbindung der Formel (I) versus Verbindung der Formel (II)

Die zu beurteilenden Verbindungen werden in geruchsneutralen, wärmebeständigen Kunststoffbeuteln vorbereitet und beurteilt.

Die Probanden bewerten die Intensität in Luft der zu beurteilenden Verbindungen auf einer Skala von 1 (geruchlos) bis 9 (sehr stark).

| | Verbindung der Formel (I) | Verbindung der Formel (II) |
|---|---|---|
| Intensität in Luft | 1 | 6 |

Die Verbindung der Formel (I) wird als geruchlos beschrieben, wohingegen die Verbindung der Formel (II) als sehr viel stärker beurteilt wird.

## Patentansprüche

1. Verbindung der Formel (II)

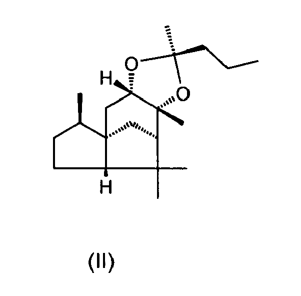

2. Mischung, umfassend
(i) Verbindung der Formel (I) 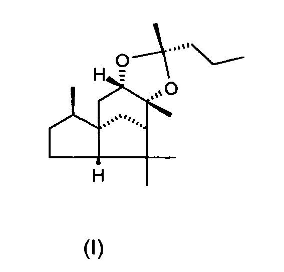 und
(ii) Verbindung der Formel (II) wie in Anspruch 1 definiert,
**dadurch gekennzeichnet, dass**
- das Gewichtsverhältnis der Verbindung der Formel (II) zu der Verbindung der Formel (I) im Bereich von 4 : 1 bis 1 : 10 liegt, vorzugsweise im Bereich von 2 : 1 bis 1 : 6, bevorzugt im Bereich von 1 : 1 bis 1 : 3,
und/oder
- der Gesamtanteil der Verbindungen der Formeln (I) und (II) zusammen größer ist als 40 Gew.-%, vorzugsweise größer als 55 Gew.-%, bevorzugt größer als 70 Gew.-%, weiter bevorzugt größer als 85 Gew.-%, besonders bevorzugt größer als 95 Gew.-%, bezogen auf die Gesamtmenge der in der Mischung enthaltenen 3,6,8,8-Tetramethyl-octahydro-3a,7-methano-azulen-5,6-diol-methyl-n-propylketale.

3. Verwendung der Verbindung (II) gemäß Anspruch 1 oder einer Mischung gemäß Anspruch 2
- als Riechstoff, vorzugsweise als Riechstoff mit den Geruchsnoten Ambra und/oder Holz,
und/oder
- als Mittel zum Erhöhen der Substantivität und/oder der Retention einer Riechstoffmischung,
und/oder
- als Fixateur.

4. Riechstoffmischung, vorzugsweise Parfümöl, umfassend
- die Verbindung der Formel (II) wie in Anspruch 1 oder eine Mischung wie in Anspruch 2 definiert,
und
- einen, 2, 3, 4, 5, 6, 7, 8, 9, 10 oder mehr weitere Riechstoffe, die keine 3,6,8,8-Tetramethyl-octahydro-3a,7-methano-azulen-5,6-diol-methyl-n-propylketale sind,
wobei
(i) die Menge an Verbindung der Formel (II) ausreicht, eine Ambranote und/oder Holznote, insbesondere eine weisse Ambranote ("white amber"), zu vermitteln,
und/oder
(ii) der oder die weiteren Riechstoffe eine, mehrere oder sämtliche der Noten fruchtig, blumig, würzig, holzig, moschus und/oder ambriert vermitteln und die Menge der Verbindung der Formel (II) ausreicht, eine oder mehrere dieser Noten zu modifizieren und/oder zu verstärken,
und/oder
(iii) die Menge an Verbindung der Formel (II) ausreicht, der Riechstoffmischung einen Eindruck von Volumen, Komplexität, Eleganz und/oder Natürlichkeit zu verleihen, und/oder
(iv) die Menge an Verbindung der Formel (II) ausreicht, um im Vergleich zu einer ansonsten identisch zusammengesetzten Vergleichsriechstoffkomposition ohne Verbindung der Formel (II), einen pflegenderen, harmonischeren, hochwertigeren und/oder natürlicheren Geruchseindruck zu bewirken.

5. Riechstoffmischung, vorzugsweise Parfümöl, nach Anspruch 4, **dadurch gekennzeichnet, dass** die Menge an Verbindung der Formel (II) wie in Anspruch 1 definiert im Bereich von 0,0001 bis 25 Gew.-% liegt, vorzugsweise im Bereich von 0,001 bis 15 Gew.-%, jeweils bezogen auf das Gesamtgewicht der Riechstoffmischung.

6. Riechstoffmischung, vorzugsweise Parfümöl, nach Anspruch 4 oder 5, umfassend
(a) einen, 2, 3 oder mehr weitere Holz- und/oder Ambra-Riechstoffe,
und/oder
(b) einen, 2, 3 oder mehr Moschusriechstoffe.

7. Riechstoffmischung, vorzugsweise Parfümöl, nach Anspruch 6, wobei der oder die Moschusriechstoffe des Bestandteils (b) gewählt sind aus der Gruppe der macrocyclischen Moschusriechstoffe, der Nitro-Moschusriechstoffe, der polycyclischen Moschusriechstoffe und/oder der alicyclischen Moschusriechstoffe.

8. Riechstoffmischung, vorzugsweise Parfümöl, nach Anspruch 6 oder 7, wobei der oder die Moschusriechstoffe des Bestandteils (b) gewählt sind aus der Gruppe der polycyclischen und/oder der macrocyclischen Moschusriechstoffe, vorzugsweise gewählt aus der Gruppe bestehend aus macrocyclischen C₁₄-C₁₈-Ketonen und macrocyclischen C₁₄-C₁₈-Lactonen.

9. Riechstoffmischung, vorzugsweise Parfümöl, nach einem der Ansprüche 5 bis 8, wobei das Massenverhältnis der Gesamtmenge an Moschusriechstoffen des Bestandteils (b) zu Verbindung (II) gleich oder größer als 10 : 1 ist, vorzugsweise im Bereich von 10 : 1 bis 100.000 : 1, weiter bevorzugt im Bereich von 50 : 1 bis 100.000 : 1, besonders bevorzugt im Bereich von 100 : 1 bis 100.000 : 1 liegt.

10. Verfahren zum Verstärken eines Geruchs mit einer der Noten fruchtig, blumig, würzig, holzig, moschus und/oder ambriert, umfassend den folgenden Schritt:
- Vermischen von
einem oder mehreren Riechstoffen mit einer, mehrerer oder sämtlichen der Noten fruchtig, blumig, würzig, holzig, moschus und/oder ambriert,
(i) mit einer Menge an Verbindung (II) wie in Anspruch 1 definiert oder einer Mischung gemäß Anspruch 2, die ausreicht, den Geruchseindruck des bzw. der Riechstoffe, die eine, mehrere oder sämtliche der Noten fruchtig, blumig, würzig, holzig, moschus und/oder ambriert verursachen, zu betonen,
oder
(ii) mit einer Riechstoffmischung, vorzugsweise einem Parfümöl, gemäß einem der Ansprüche 4 bis 9, in einer Menge, die ausreicht, den Geruchseindruck des bzw. der Riechstoffe, die eine, mehrere oder sämtliche der Noten fruchtig, blumig, würzig, holzig, moschus und/oder ambriert verursachen, zu betonen.

11. Parfümiertes Produkt enthaltend Verbindung (II) gemäß Anspruch 1, eine Mischung gemäß Anspruch 2 oder eine Riechstoffmischung nach einem der Ansprüche 4 bis 9, vorzugsweise in einer sensorisch wirksamen Menge.

12. Parfümiertes Produkt nach Anspruch 11, **dadurch gekennzeichnet, dass** das Produkt ein oder mehrere Tenside enthält.

13. Parfümiertes Produkt nach Anspruch 11 oder 12, ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalts.

14. Verfahren zum Versehen von (a) Haaren oder (b) textilen Fasern mit einer, mehrerer oder sämtlicher der Geruchsnoten fruchtig, blumig, würzig, holzig, moschus und/oder ambriert mit folgenden Schritten:
- Bereitstellen einer Riechstoffmischung, vorzugsweise nach einem der Ansprüche 4 bis 9, umfassend
(a) Verbindung (II) wie in Anspruch 1 definiert oder eine Mischung gemäß Anspruch 2 und
(b) einen oder mehrere weitere Riechstoffe, der bzw. die eine, mehrere oder sämtliche der Noten fruchtig, blumig, würzig, holzig, moschus und/oder ambriert vermitteln, wobei die Menge an Verbindung (II) ausreicht, eine oder mehrere dieser Noten zu modifizieren und/oder zu verstärken,
- Applizieren der Mischung auf (a) das Haar oder (b) die textilen Fasern.

## Claims

1. Compound of Formula (II):

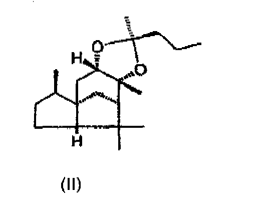

2. Mixture, comprising
(i) compound of Formula (I) 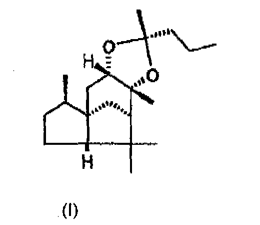 and
(ii) compound of Formula (II) as defined in Claim 1,
**characterised in that**
- the weight ratio of the compound of Formula (II) to the compound of Formula (I) lies in the range of from 4 : 1 to 1 : 10, preferably in the range of from 2 : 1 to 1 : 6, preferably in the range from 1 : 1 to 1 : 3,
and/or
- the total proportion of the compounds of Formulae (I) and (II) together is greater than 40 wt.%, preferably greater than 55 wt.%, preferably greater than 70 wt.%, more preferably greater than 85 wt.%, particularly preferably greater than 95 wt.%, expressed in terms of the total amount of the 3,6,8,8-tetramethyl-octahydro-3a,7-methano-azulen-5,6-dinl-methyl-n-propylketals contained in the mixture.

3. Use of the compound (II) according to Claim 1 or a mixture according to Claim 2
- as a fragrance, preferably as a fragrance with the aroma of amber and/or wood,
and/or
- as means for increasing the substantivity and/or the retention of a fragrance mixture,
and/or
- as fixative.

4. Fragrance mixture, preferably perfumed oil, comprising
- the compound of Formula (II) as defined in Claim 1 or a mixture as defined in Claim 2.
and
- one, 2, 3, 4, 5, 6, 7, 8, 9, 10 or more further fragrances which are not 3,6,8,8-tetramethyl-octahydro-3a,7-methano-azulen-5,6-diol-methyl-n-propylketals, wherein
(i) the amount of compound of Formula (II) is sufficient to impart an amber note and/or wood note, in particular a white amber note,
and/or
(ii) the further fragrance or fragrances impart one, a plurality of or all of the notes fruity, flowery, spicy, woody, musk and/or amber, and the amount of compound of Formula (II) is sufficient to modify and/or enhance one or a plurality of these notes,
and/or
(iii) the amount of compound of Formula (II) is sufficient to give the fragrance mixture an impression of volume, complexity, elegance and/or naturalness, and/or
(iv) the amount of compound of Formula (II) is sufficient, in comparison with an otherwise identically composed reference fragrance composition without compound of Formula (II), to cause a gentler, more harmonic, higher-quality and/or more natural odour impression.

5. Fragrance mixture, preferably perfumed oil, according to Claim 4, **characterised in that** the amount of compound of Formula (II) as defined in Claim 1 lies in the range from 0.0001 to 25 wt.%, preferably in the range of from 0.001 to 15 wt.%, in each case expressed in terms of the total weight of the fragrance mixture.

6. Fragrance mixture, preferably perfumed oil, according to Claim 4 or 5, comprising
(a) one, 2, 3 or more further wood and/or amber fragrances,
and/or
(b) one, 2, 3 or more musk fragrances.

7. Fragrance mixture, preferably perfumed oil, according to Claim 6, wherein the musk fragrance or fragrances of component (b) are selected from the group of macrocyclic musk fragrances, nitro-musk fragrances, polycyclic musk fragrances and/or alicyclic musk fragrances.

8. Fragrance mixture, preferably perfumed oil, according to Claim 6 or 7, wherein the musk fragrance or fragrances of component (b) are selected from the group of polycyclic and/or macrocyclic musk fragrances, preferably selected from the group consisting of macrocyclic C₁₄-C₁₈ ketones and macrocyclic C₁₄-C₁₈ lactones,

9. Fragrance mixture, preferably perfumed oil, according to any one of Claims 5 to 8, wherein the mass ratio of the total amount of musk fragrances of component (b) to compound (II) is greater than or equal to 10 : 1, preferably in the range of from 10 : 1 to 100,000 : 1, more preferably in the range from 50 : 1 to 100,000 : 1, particularly preferably in the range from 100 : 1 to 100,000 : 1.

10. Method for enhancing an odour having one of the notes fruity, flowery, spicy, woody, musk and/or amber, comprising the following steps:
- mixing
one or a plurality of fragrances having one, a plurality of or all of the notes fruity, flowery, spicy, woody, musk and/or amber,
(i) with an amount of compound (II) as defined in Claim 1 or a mixture according to Claim 2 which is sufficient to tone the odour impression of the fragrance or fragrances which cause one, a plurality of or all of the notes fruity, flowery, spicy, woody, musk and/or amber, or
(ii) with a fragrance mixture, preferably a perfumed oil, according to any one of Claims 4 to 9 in an amount which is sufficient to tone the odour impression of the fragrance or fragrances which cause one, a plurality of or all of the notes fruity, flowery, spicy, woody, musk and/or amber.

11. Perfumed product containing compound (II) according to Claim 1, a mixture according to Claim 2 or a fragrance mixture according to any one of Claims 4 to 9, preferably in a sensorially effective amount.

12. Perfumed product according to Claim 11, **characterised in that** the product contains one or a plurality of surfactants.

13. Perfumed product according to Claim 11 or 12, selected from the group consisting of washing and cleaning agents, hygiene or care products, in particular from the field of body and hair care, cosmetics and household.

14. Method for providing (a) hair or (b) textile fibres with one, a plurality of or all of the notes fruity, flowery, spicy, woody, musk and/or amber, having the following steps:
- providing an odour mixture, preferably according to any one of Claims 4 to 9, comprising
(a) compound (II) as defined in Claim 1 or a mixture according to Claim 2 and
(b) one or a plurality of further fragrances which impart one, a plurality of or all of the notes fruity, flowery, spicy, woody, musk and/or amber, the amount of compound (II) being sufficient to modify and/or enhance one or a plurality of these notes,
- applying the mixture to (a) the hair or (b) the textile fibres.

## Revendications

1. Composé de formule (II)

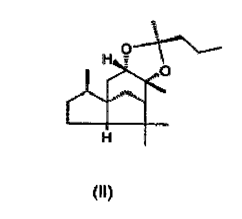

2. Mélange comprenant
(i) un composé de formule (I) 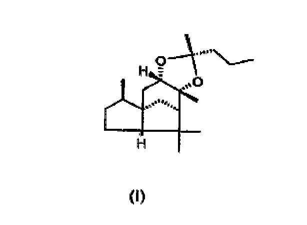 et
(ii) un composé de formule (II) tel que défini dans la revendication 1,
**caractérisé en ce que**
- le rapport en poids du composé de formule (II) au composé de formule (I) se situe dans une plage de 4 : 1 à 1 : 10, de préférence dans une plage de 2 : 1 à 1 : 6, de manière préférée dans une plage de 1 : 1 à 1 : 3, et/ou
- la proportion totale des composés de formules (I) et (II) conjointement est supérieure à 40 % en poids, de préférence supérieure à 55 en poids, de préférence supérieure à 70 % en poids, de manière davantage préférée, supérieure à 85 % en poids, de manière particulièrement préférée, supérieure à 95 % en poids, par rapport à la quantité totale de 3,6,8,8-tétraméthyloctahydro-3a,7-méthane-azulèn-5,6-diol-méthyl-n-propylcétal.

3. Utilisation du composé (II) selon la revendication 1 ou d'un mélange selon la revendication 2
- comme substance odoriférante, de préférence comme substance odoriférante ayant des notes olfactives ambrées et/ou boisées,
et/ou
- comme agent pour augmenter la substantivité et/ou la rétention d'un mélange de substances odoriférantes
et/ou
- comme fixateur.

4. Mélange de substances odoriférantes, de préférence des huiles de parfum, comprenant
- le composé de formule (II) selon la revendication 1 ou un mélange selon la revendication 2, et
- une, 2, 3, 4, 5, 6, 7, 8, 9, 10 autres substances odoriférantes ou plus, qui ne sont pas le 3,6,8,8-tétraméthyloctahydro-3a,7-méthano-azulèn-5,6-diol-méthyl-n-propylcétal,
dans lequel
(i) la quantité de composé de formule (II) suffit pour conférer une note ambrée et/ou une note boisée, en particulier, une note d'ambre blanc ("white amber"),
et/ou
(ii) la ou les autres substances odoriférantes confèrent une, plusieurs ou toutes les notes fruitées, fleuries, épicées, boisées, musquées et/ou ambrées et la quantité de composé de formule (II) suffit pour modifier et/ou renforcer une ou plusieurs de ces notes,
et/ou
(iii) la quantité de composé de formule (II) suffit pour prêter au mélange de substances odoriférantes une impression de volume, de complexité, d'élégance et/ou de naturel,
et/ou
(iv) la quantité de composé de formule (II) suffit pour entraîner comparativement à une composition de substances odoriférantes comparatives sans composé de formule (II), par ailleurs identique, une impression olfactive accrue de soin, d'harmonie, de préciosité et/ou de naturel.

5. Mélange de substances odoriférantes, de préférence des huiles de parfum, selon la revendication 4, **caractérisé en ce que** la quantité de composé de formule (II) telle que définie dans la revendication 1 se situe dans une plage de 0,0001 à 25 % en poids, de préférence dans une plage de 0,001 à 15 % en poids, respectivement par rapport au poids total du mélange de substances odoriférantes.

6. Mélange de substances odoriférantes, de préférence des huiles de parfum, selon la revendication 4 ou 5, comprenant
(a) un, 2, 3 substances odoriférantes boisées et/ou ambrées, ou plus
et/ou
(b) une, 2, 3 substances odoriférantes musquées, ou plus.

7. Mélange de substances odoriférantes, de préférence des huiles de parfum, selon la revendication 6, dans lequel la ou les substances odoriférantes musquées du composant (b) sont choisies dans le groupe des substances odoriférantes musquées macrocycliques, des substances odoriférantes musquées nitro, des substances odoriférantes musquées polycycliques et/ou des substances odoriférantes musquées alicycliques.

8. Mélange de substances odoriférantes, de préférence des huiles de parfum, selon la revendication 6 ou 7, dans lequel la ou les substances odoriférantes musquées du composant (b) sont choisies dans le groupe des substances odoriférantes musquées polycycliques et/ou macrocycliques, de préférence choisies dans le groupe comprenant des cétones en C₁₄ à C₁₈ macrocycliques et des lactones en C₁₄ à C₁₈ macrocycliques.

9. Mélange de substances odoriférantes, de préférence des huiles de parfum, selon l'une des revendications 5 à 8, dans lequel le rapport en poids de la quantité totale de substances odoriférantes musquées du composant (b) au composé (II) est supérieur ou égal à 10 : 1, de préférence dans une plage de 10 : 1 à 100.000 : 1, de manière davantage préférée dans une plage de 50 : 1 à 100.000 : 1, de manière particulièrement préférée, dans une plage de 100 : 1 à 100.000 : 1.

10. Procédé de renforcement d'une odeur présentant les notes fruitées, fleuries, épicées, boisées, musquées et/ou ambrées, comprenant l'étape suivante :
- mélange
d'une ou plusieurs substances odoriférantes présentant une, plusieurs ou toutes les notes fruitées, fleuries, épicées, boisées, musquées et/ou ambrées,
(i) avec une quantité de composé (II) tel que défini dans la revendication 1 ou avec un mélange selon la revendication 2, qui suffit pour souligner l'impression olfactive de la ou des substances odoriférantes qui entraînent une, plusieurs ou toutes les notes fruitées, fleuries, épicées, boisées, musquées et/ou ambrées,
ou
(ii) avec un mélange de substances odoriférantes, de préférence une huile de parfum, selon l'une des revendications 4 à 9, en une quantité qui suffit pour souligner l'impression olfactive de la ou des substances odoriférantes qui entraînent une, plusieurs ou toutes les notes fruitées, fleuries, épicées, boisées, musquées et/ou ambrées.

11. Produit parfumé contenant un composé (II) selon la revendication 1, un mélange selon la revendication 2 ou un mélange de substances odoriférantes selon l'une des revendications 4 à 9, de préférence en une quantité efficace du point de vue sensoriel.

12. Produit parfumé selon la revendication 11, **caractérisé en ce que** le produit contient un ou plusieurs tensioactifs.

13. Produit parfumé selon la revendication 11 ou 12, choisi dans le groupe comprenant les agents lavants et nettoyants, les produits d'hygiène et de soin, en particulier dans le domaine des soins du corps et des cheveux, des cosmétiques et de l'entretien domestique.

14. Procédé permettant de doter (a) les cheveux ou (b) les fibres textiles d'une, plusieurs ou toutes les notes olfactives fruitées, fleuries, épicées, boisées, musquées et/ou ambrées, comportant les étapes suivantes :
- la mise à disposition d'un mélange de substances odoriférantes, de préférence selon l'une des revendications 4 à 9, comprenant
(a) le composé (II) tel que défini dans la revendication 1 ou un mélange selon la revendication 2 et
(b) une ou plusieurs autres substances odoriférantes qui confèrent une, plusieurs ou toutes les notes fruitées, fleuries, épicées, boisées, musquées et/ou ambrées, la quantité de composé (II) suffisant pour modifier et/ou renforcer une ou plusieurs de ces notes,
- l'application du mélange de (a) sur les cheveux ou de (b) sur les fibres textiles.
